# FASCICULE DE BREVET EUROPEEN

(11) **EP 0 689 595 B1**
(45) Date de publication et mention de la délivrance du brevet: **15.10.2003**
(21) Numéro de dépôt: 94911205.6
(22) Date de dépôt: 23.03.1994
(51) Int. Cl.: C12N 15/29, C12N 15/82, C12Q 1/68, A01N 65/00, C12N 5/10, A01H 5/00

(54) **PROMOTEUR VEGETAL, MICROORGANISMES ET CELLULES VEGETALES CONTENANT UNE UNITE D'EXPRESSION D'UNE PROTEINE D'INTERET COMPRENANT LEDIT PROMOTEUR**
PFLANZENPROMOTER, MICROORGANISMEN UND PFLANZENZELLEN MIT EINER EXPRESSIONSEINHEIT FÜR EIN GEWÜNSCHTES PROTEIN, DAS BESAGTEN PROMOTER ENTHÄLT
PLANT PROMOTER, MICROORGANISMS AND PLANT CELLS CONTAINING A UNIT FOR THE EXPRESSION OF A PROTEIN OF INTEREST COMPRISING SAID PROMOTER

(30) Priorité: 23.03.1993 FR 9303299
(43) Date de publication de la demande: 03.01.1996
(73) Titulaire: Biogemma, 75001 Paris (FR)
(72) Inventeur: MARCO, Yves, F-31320 Castanet-Tolosan (FR); ROBY, Dominique, F-31450 POMPERTUZAT (FR); SCHNEIDER, Michel, CH-1201 GENEVE (CH); TOPPAN, Alain, F-31700 Cornebarrieu (FR)
(74) Mandataire: Gillard, Marie-Louise
(86) Numéro de dépôt international: FR9400316
(87) Numéro de publication internationale: WO94021793

(56) Documents cités:
- WO-A-91/15585
- PROCEEDINGS OF THE NATIONAL ACADEMY OF SCIENCES OF USA vol. 87 , Octobre 1990 , WASHINGTON US pages 8013 - 8016 TAKAHASHI, Y., ET AL. 'Location of the cis-acting auxin-responsive region in the promoter of the par gene from tobacco mesophyll protoplasts'
- MOLECULAR AND GENERAL GENETICS vol. 236, no. 2/3 , Janvier 1993 , BERLIN DE pages 179 - 186 MARTINI, N., ET AL. 'Promoter sequences of a potato pathogenesis-related gene mediate transcriptional activation selectively upon fungal infection'
- BIOTECHNOLOGY vol. 8, no. 9 , Septembre 1990 , NEW YORK US pages 845 - 848 DOERNER, P.W., ET AL. 'Plant defense gene promoter-reporter gene fusions in transgenic plants: tools for identification of novel inducers'
- PLANT MOLECULAR BIOLOGY. vol. 17 , 1991 , DORDRECHT, THE NETHERLANDS. pages 409 - 413 GODIARD, L., ET AL. 'Differential regulation in tobacco cell suspensions of genes involved in plant-bacteria interactions by pathogen-related signals'
- PLANT MOLECULAR BIOLOGY. vol. 15 , 1990 , DORDRECHT, THE NETHERLANDS. pages 145 - 154 MARCO, Y.J., ET AL. 'Transcriptional activation of 2 classes of genes during the hypersensitive reaction of tobacco leaves infiltrated with an incompatible isolate of the phytopathogenic bacterium Pseudomonas solanacearum'

### LISTE DE SEQUENCES

(1) INFORMATIONS GENERALES:
   (i) DEPOSANT:
      (A) NOM : BIOGEMMA
      (B) RUE: 1, rue Edouard Colonne
      (C) VILLE : PARIS
      (E) PAYS : France
      (F) CODE POSTAL : 75001
      (G) TELEPHONE : 01 55 34 94 06
   (ii) TITRE DE L' INVENTION: PROMOTEUR VEGETAL, MICROORGANISMES ET CELLULES VEGETALES CONTENANT UNE UNITE D'EXPRESSION D'UNE PROTEINE D'INTERET COMPRENANT LEDIT PROMOTEUR
   (lii) NOMBRE DE SEQUENCES: 18
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 631 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (vii) SOURCE IMMEDIATE:
      (B) CLONE: 246
   (ix) CARACTERISTIQUE ADDITIONELLE:
      (A) NOM/CLE CDS
      (B) EMPLACEMENT: join(1..177, 368..631)
   (ix) CARACTERISTIQUE ADDITIONELLE:
      (A) NOM/CLE: intron
      (B) EMPLACEMENT: 178..367
   (ix) CARACTERISTIQUE ADDITIONELLE:
      (A) NOM/CLE: mise-signal
      (B) EMPLACEMENT: 175..182
      (D) AUTRES RENSEIGNEMENTS: /fonction= "séquences consensus d'épissage"
   (ix) CARACTERISTIQUE ADDITIONELLE:
      (A) NOM/CLE: misc-signal
      (B) EMPLACEMENT: 323..333
      (D) AUTRES RENSEIGNEMENTS: /fonction= "séquences consensus d'épissage"
   (ix) CARACTERISTIQUE ADDITIONELLE:
      (A) NOM/CLE: mise-signal
      (B) EMPLACEMENT: 363..368
      (D) AUTRES RENSEIGNEMENTS: /fonction= "séquence consensus d'épissage"
   (xi) DESCRIPTION DE LA SEQUENCE: SEQ ID NO: 1:
   ATG AAC CCT GTT CAC AAA AAG ATC CCT ATT TTG ATT CAC AAT AGT AAA      48
   Met Asn Pro Val His Lys Lys Ile Pro Ile Leu Ile His Asn Ser Lys
     1               5                  10                  15
   GCC ATT TGT GAG TCT CTA AAC ATT CTT GAG TAC ATT GAT GAA GTC TGG      96
   Ala Ile Cys Glu Ser Leu Asn Ile Leu Glu Tyr Ile Asp Glu Val Trp
                20                  25                  30
   CAT GAC AAA TGT CCA TTA CTT CCT TCT GAT CCT TAC GAA AAG TCA CAA     144
   His Asp Lys Cys Pro Leu Leu Pro Ser Asp Pro Tyr Glu Lys Ser Gln
            35                  40                  45
   GCC AGA TTC TGG GCC GAC TAT ATT GAC AAG AAG GTAATAAACA TCTCACAAAG    197
   Ala Arg Phe Trp Ala Asp Tyr Ile Asp Lys Lys
        50                  55
   ACTTAACAGT CAATGTAACA TGACCTTTAC TAAGTTCATC TTGTGTAGTT TCACCGAGCT    257
   GTTTAAGGTC GTCGTACATT TGAATATTAG GTGTTTCACA TTTGAATTTT TTTATCCCCT    317
   TGTTAGAATT CCTGATTCTG TCAATACTTA TGGACGTTGG TTTAATGCAG ATA TAT       373
                                                         Ile Tyr
                                                          60
   AGC ACA GGA AGA AGA GTG TGG AGC GGT AAA GGT GAA GAT CAA GAA GAA    421
   Ser Thr Gly Arg Arg Val Trp Ser Gly Lys Gly Glu Asp Gln Glu Glu
               65                  70                  75
   GCA AAG AAG GAA TTC ATA GAA ATA CTC AAG ACT TTG GAA GGA GAG CTT    469
   Ala Lys Lys Glu Phe Ile Glu Ile Leu Lys Thr Leu Glu Gly Glu Leu
           80                  85                  90
   GGA AAT AAA ACT TAC TTT GGT GGT GAT AAT CTG GGT TTT GTG GAT GTG    517
   Gly Asn Lys Thr Tyr Phe Gly Gly Asp Asn Leu Gly Phe Val Asp Val
       95                  100                 105
   GCT TTG GTT CCC TTT ACT AGT TGG TTT TAT TCT TAT GAG ACT TGT GCA    565
   Ala Leu Val Pro Phe Thr Ser Trp Phe Tyr Ser Tyr Glu Thr Cys Ala
   110                 115                 120                 125
   AAC TTT AGT ATA GAA GCA GAG TGT CCA AAG CTG GTG GTA TGG GCA AAA    613
   Asn Phe Ser Ile Glu Ala Glu Cys Pro Lys Leu Val Val Trp Ala Lys
                   130                 135                 140
   ACA TGT ATG GAG AGC GAG                                            631
   Thr Cys Met Glu Ser Glu
               145
(2) INFORMATION POUR LA SEQ ID NO: 2:
   (i) CARACTERISTIQUES DE LA SEQUENCE:
      (A) LONGUEUR: 147 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Met Asn Pro Val His Lys Lys Ile Pro Ile Leu Ile His Asn Ser Lys
   1 5 10 15
   Ala Ile Cys Glu Ser Leu Asn Ile Leu Glu Tyr Ile Asp Glu Val Trp
   20 25 30
   His Asp Lys Cys Pro Leu Leu Pro Ser Asp Pro Tyr Glu Lys Ser Gln
   35 40 45
   Ala Arg Phe Trp Ala Asp Tyr Ile Asp Lys Lys Ile Tyr Ser Thr Gly
   50 55 60
   Arg Arg Val Trp Ser Gly Lys Gly Glu Asp Gln Glu Glu Ala Lys Lys
   65 70 75 80
   Glu Phe Ile Glu Ile Leu Lys Thr Leu Glu Gly Glu Leu Gly Asn Lys
   85 90 95
   Thr Tyr Phe Gly Gly Asp Asn Leu Gly Phe Val Asp Val Ala Leu Val
   100 105 110
   Pro Phe Thr Ser Trp Phe Tyr Ser Tyr Glu Thr Cys Ala Asn Phe Ser
   115 120 125
   Ile Glu Ala Glu Cys Pro Lys Leu Val Val Trp Ala Lys Thr Cys Met
   130 135 140
   Glu Ser Glu
   145
(2) INFORMATION POUR LA SEQ ID NO: 3:
   (i) CARACTERISTIQUES DE LA SEQUENCE:
      (A) LONGUEUR: 441 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (vii) SOURCE IMMEDIATE:
      (B) CLONE: 246
   (xi) DESCRIPTION DE LA SEQUENCE; SEQ ID NO: 3:
   ATGAACCCTG TTCACAAAAA GATCCCTATT TTGATTCACA ATAGTAAAGC CATTTGTGAG     60
   TCTCTAAACA TTCTTGAGTA CATTGATGAA GTCTGGCATG ACAAATGTCC ATTACTTCCT    120
   TCTGATCCTT ACGAAAGGTC ACAAGCCAGA TTCTGGGCCG ACTATATTGA CAAGAAGATA    180
   TATAGCACAG GAAGAAGAGT GTGGAGCGGT AAAGGTGAAG ATCAAGAAGA AGCAAAGAAG    240
   GAATTCATAG AAATACTCAA GACTTTGGAA GGAGAGCTTG GAAATAAAAC TTACTTTGGT    300
   GGTGATAATC TGGGTTTTGT GGATGTGGCT TTGGTTCCCT TTACTAGTTG GTTTTATTCT    360
   TATGAGACTT GTGCAAACTT TAGTATAGAA GCAGAGTGTC CAAAGCTGGT GGTATGGGCA    420
   AAAACATGTA TGGAGAGCGA G                                              441
(2) INFORMATION POUR LA SEQ ID NO: 4:
   (i) CARACTERISTIQUES DE LA SEQUENCE:
      (A) LONGUEUR: 1096 paires de bases
      (B) TYPE: acide nucléique
      (C) NOMBRE DE BRINS: simple
      (D) CONFIGURATION: linéaire
   (ii) TYPE DE MOLECULE: ADN (génonique)
   (iii) HYPOTHETIQUE: NON
   (xi) DESCRIPTION DE LA SEQUENCE: SEQ ID NO: 4:
   TCAAATGAAA TACACATAAG AAGCACATAA ATTTAAATGC CGTATTAAAC TTACAGTATA     60
   CTATAGCGGA AGTTGGCTTG ATAAAGGAAC GCTGAGGAGA GTAGCCGATG GTGAAACACT    120
   AACATCAAGT GCAAAAGAAA GAAAAACTGA AAACAGAAGA TGAATGTTTG AAGTGGGTAA    180
   AAGATTACTT AAAAGATAGG TTTGGTTAAC AAATGATTGT GACTGTTACG AAGCAGTGTG    240
   AACCGTTGGG ACTTTTAATA TTCTTCGGCA GAAGAACATT GCTCTTTCCA CGTATGTAGT    300
   CTTTGTCTAC TTGTAGTTTT TTTTAATTTA AATTAAATAA GTTAATTAGA GAAATAATAA    360
   GAAGGATATT TTAGTAATTC AACTTTTAAC TTTTAGGTTT CCCACTTATA ATATAATATA    420
   GATATAGTTT TTTTTAATTT AAATTAAATA AGTTAATTAG AGAAATAATA AGAAGGATAT    480
   TTTAGTAATT CAACTTTTAA CTTTTAGGGT TTCCACTTAT AATATAATAT AGATATAGAT    540
   ATAGATATAG ATATAGATAA AGATATATAG ATATAGATAG ATAATATAGA TGGATGAGTC    600
   ATTGGCGATA AAGTGAGGAT TGTTTCATTT TTGTTATTAA AAACTTACTA CTCCTTAAAT    660
   ATAAAATATG ATTCCTTTTA AAAAAGAAAT AGAATAAAAA TAAAGATAAA ACACTAAAAA    720
   TAAATTAATT GTCTAGACAA AATCTACCGT TCACCTCAAT TAATACACAT CCCCGTCCAC    780
   ATCATGAAGT AGCTAGCACA AGCGTACAGA TCAGTTGAAA GAAGAAAAGG GTCCAGTCCT    840
   AAATATCCAA ATGTTCATGA AAGGAGGACA ACTTAGTTTT TTCTACTAGA AAGAATATTT    900
   TGACGAATTT CGTTCACATT GGCATGCTTT AATTATATTA AGTAGTCTTT CTTGGAAAAG    960
   AAGTATTTGC AATATCAAAC CAAATCTTCC CATTACGCAA GCAATGACAT CTAAGCAAAT   1020
   ATATATCACT ATAAATAGTA CTACTAATGT TCAATGACTT TTATAAGCAC TACATATATA   1080
   TACTCAAACA AAAAGA                                                   1096
(2) INFORMATION POUR LA SEQ ID NO: 5:
   (i) CARACTERISTIQUES DE LA SEQUENCE:
      (A) LONGUEUR: 236 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (xi) DESCRIPTION DE LA SEQUENCE: SEQ ID NO: 5:
   CCTTTTTCGA TTCTAATCCA ATCAATTCAA CAGTGTAAGG TGAAGCAGTC AATTTAAAGG    60
   AAGGCCTTTA AATTCTAAAA TATTGTACTT TTCCTGCGCT TCTAAAAGTG AACGACAAAG   120
   AAAAAATAGT TATTCTTGAA CTTAATATTG TACAATAGGA TAAATTTTAA CTATCTATAA   180
   AAAGAGAACA AAACCTTAAT CTCTTCAAAA TAATATTATA AGAAGTAACA TAATTG       236
(2) INFORMATION POUR LA SEQ ID NO: 6:
   (i) CARACTERISTIQUES DE LA SEQUENCE:
      (A) LONGUEUR: 813 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (xi) DESCRIPTION DE LA SEQUENCE: SEQ ID NO: 6:
   GAGCTCGGCA AGGCTGACCA AAGTCACAGA AGCGATTGGA ATTCGCAGGA CAGACCATGC    60
   ACCTGCGCAC AAAATGTCGT AGGTGCGACA CACCAGAACC AGCACTGGGC AGCAGGTTTC   120
   AATTGCTCTG TGGCTCGTTT GAAACTCATC CGAGCCACTC ATGACCTCGT CCGAATATTT   180
   CAACAAGTCC ATAAACATAA TACGGACATA CTCGGGGTTT CACTTCACGT CAAACAACAT   240
   CAAAATTACA AATCACACCC CGATTCGAAC CTTGAGTTTT AAACTTTTCA ATTTGCAAAT   300
   CTCGTGCCAA AACATATTAA ATGAATCCGG AATGACTTCA AATTTATAAA TGACATAACG   360
   GAGTTGTTCA AATTTCCAGA ATCAGATTCT GCCTTTGATA TCAAAAAGTC AACCCCGTGA   420
   TCAAACTTGG AATTCTTTAG CCTTTAAATT GCTAGTTTTC GTTAAATGGT CATAACTTGA   480
   GCTATGGACC TCCAAATTAA ATTTCGGGCA TACGCTCAAA TCCCAATTAC GAATACGGAG   540
   CTACCGGACT GTCAAAATAC TGATCCGGGT CCGTTTGCTA AAAACGTTGA CCAAAGTCCA   600
   CTAAGTTGAG TTTTAAAACT TTATTTCACA TTTTAATCCA TTTTTTACAT GAAAACTTTC   660
   CGGAAAATAC GGAGTATGCA CGCAAGTCGA GGAATGATAA ATGGTACGTT TCGAAGTTTT   720
   AGAACTCAAA ATTACTTATT AAATTTAAAG ATGACATTTT GGGTCATCAC ATTGATGAAA   780
   ATTTTGACAT TAATATCTGA GAACTTTCTT TGA                                813
(2) INFORMATION POUR LA SEQ ID NO: 7:
   (i) CARACTERISTIQUES DE LA SEQUENCE:
      (A) LONGUEUR: 3046 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (vii) SOURCE IMMEDIATE:
      (B) CLONE: 246C
   (xi) DESCRIPTION DE LA SEQUENCE: SEQ ID NO: 7:
   GAGCTCGGCA AGGCTGACCA AAGTCACAGA AGCGATTGGA ATTCGCAGGA CAGACCATGC 60
   ACCTGCGCAC AAAATGTCGT AGGTGCGACA CACCAGAACC AGCACTGGGC AGCAGGTTTC 120
   AATTGCTCTG TGGCTCGTTT GAAACTCATC CGAGCCACTC ATGACCTCGT CCGAATATTT 180
   CAACAAGTCC ATAAACATAA TACGGACATA CTCGGGGTTT CACTTCACGT CAAACAACAT 240
   CAAAATTACA AATCACACCC CGATTCGAAC CTTGAGTTTT AAACTTTTCA ATTTGCAAAT 300
   CTCGTGCCAA AACATATTAA ATGAATCCGG AATGACTTCA AATTTATAAA TGACATAACG 360
   GAGTTGTTCA AATTTCCAGA ATCAGATTCT GCCTTTGATA TCAAAAAGTC AACCCCGTGA 420
   TCAAACTTGG AATTCTTTAG CCTTTAAATT GCTAGTTTTC GTTAAATGGT CATAACTTGA 480
   GCTATGGACC TCCAAATTAA ATTTCGGGCA TACGCTCAAA TCCCAATTAC GAATACGGAG 540
   CTACCGGACT GTCAAAATAC TGATCCGGGT CCGTTTGCTA AAAACGTTGA CCAAAGTCCA 600
   CTAAGTTGAG TTTTAAAACT TTATTTCACA TTTTAATCCA TTTTTTACAT GAAAACTTTC 660
   CGGAAAATAC GGAGTATGCA CGCAAGTCGA GGAATGATAA ATGGTACGTT TCGAAGTTTT 720
   AGAACTCAAA ATTACTTATT AAATTTAAAG ATGACATTTT GGGTCATCAC ATTGATGAAA 780
   ATTTTGACAT TAATATCTGA GAACTTTCTT TGACCTTTTT CGATTCTAAT CCAATCAATT 840
   CAACAGTGTA AGGTGAAGCA GTCAATTTAA AGGAAGGCCT TTAAATTCTA AAATATTGTA 900
   CTTTTCCTGC GCTTCTAAAA GTGAACGACA AAGAAAAAAT AGTTATTCTT GAACTTAATA 960
   TTGTACAATA GGATAAATTT TAACTATCTA TAAAAAGAGA ACAAAACCTT AATCTCTTCA 1020
   AAATAATATT ATAAGAAGTA ACATAATTGT CAAATGAAAT ACACATAAGA AGCACATAAA 1080
   TTTAAATGCC GTATTAAACT TACAGTATAC TATAGCGGAA GTTGGCTTGA TAAAGGAACG 1140
   CTGAGGAGAG TAGCCGATGG TGAAACACTA ACATCAAGTG CAAAAGAAAG AAAAACTGAA 1200
   AACAGAAGAT GAATGTTTGA AGTGGGTAAA AGATTACTTA AAAGATAGGT TTGGTTAACA 1260
   AATGATTGTG ACTGTTACGA AGCAGTGTGA ACCGTTGGGA CTTTTAATAT TCTTCGGCAG 1320
   AAGAACATTG CTCTTTCCAC GTATGTAGTC TTTGTCTACT TGTAGTTTTT TTTAATTTAA 1380
   ATTAAATAAG TTAATTAGAG AAATAATAAG AAGGATATTT TAGTAATTCA ACTTTTAACT 1440
   TTTAGGTTTC CCACTTATAA TATAATATAG ATATAGTTTT TTTTAATTTA AATTAAATAA 1500
   GTTAATTAGA GAAATAATAA GAAGGATATT TTAGTAATTC AACTTTTAAC TTTTAGGGTT 1560
   TCCACTTATA ATATAATATA GATATAGATA TAGATATAGA TATAGATAAA GATATATAGA 1620
   TATAGATAGA TAATATAGAT GGATGAGTCA TTGGCGATAA AGTGAGGATT GTTTCATTTT 1680
   TGTTATTAAA AACTTACTAC TCCTTAAATA TAAAATATGA TTCCTTTTAA AAAAGAAATA 1740
   GAATAAAAAT AAAGATAAAA CACTAAAAAT AAATTAATTG TCTAGACAAA ATCTACCGTT 1800
   CACCTCAATT AATACACATC CCCGTCCACA TCATGAAGTA GCTAGCACAA GCGTACAGAT 1860
   CAGTTGAAAG AAGAAAAGGG TCCAGTCCTA AATATCCAAA TGTTCATGAA AGGAGGACAA 1920
   CTTAGTTTTT TCTACTAGAA AGAATATTTT GACGAATTTC GTTCACATTG GCATGCTTTA 1980
   ATTATATTAA GTAGTCTTTC TTGGAAAAGA AGTATTTGCA ATATCAAACC AAATCTTCCC 2040
   ATTACGCAAG CAATGACATC TAAGCAAATA TATATCACTA TAAATAGTAC TACTAATGTT 2100
   CAATGACTTT TATAAGCACT ACATATATAT ACTCAAACAA AAAGAATGGA GAGCAACAAC 2160
   GTGGTTCTGC TAGATTTCTG GCCAAGCTCT TTTGGTATGA GGCTAAGAAT TGCATTGGCC 2220
   TTAAAGGGAA TCAAATATGA AGCAAAGGAG GAAAACTTAT CTGATAAAAG CCCTTTGCTT 2280
   CTGGAGATGA ACCCTGTTCA CAAAAAGATC CCTATTTTGA TTCACAATAG TAAAGCCATT   2340
   TGTGAGTCTC TAAACATTCT TGAGTACATT GATGAAGTCT GGCATGACAA ATGTCCATTA   2400
   CTTCCTTCTG ATCCTTACGA AAGGTCACAA GCCAGATTCT GGGCCGACTA TATTGACAAG   2460
   AAGGTAATAA ACATCTCACA AAGACTTAAC AGTCAATGTA ACATGACCTT TACTAAGTTC   2520
   ATCTTGTGTA GTTTCACCGA GCTGTTTAAG GTCGTCGTAC ATTTGAATAT TAGGTGTTTC   2580
   ACATTTGAAT TTTTTTATCC CCTTGTTAGA ATTCCTGATT CTGTCAATAC TTATGGACGT   2640
   TGGTTTAATG CAGATATATA GCACAGGAAG AAGAGTGTGG AGCGGTAAAG GTGAAGATCA   2700
   AGAAGAAGCA AAGAAGGAAT TCATAGAAAT ACTCAAGACT TTGGAAGGAG AGCTTGGAAA   2760
   TAAAACTTAC TTTGGTGGTG ATAATCTGGG TTTTGTGGAT GTGGCTTTGG TTCCCTTTAC   2820
   TAGTTGGTTT TATTCTTATG AGACTTGTGC AAACTTTAGT ATAGAAGCAG AGTGTCCAAA   2880
   GCTGGTGGTA TGGGCAAAAA CATGTATGGA GAGCGAGAGT GTCTCAAAGT CCCTTCCTCA   2940
   TCCTCACAAG ATCTATGGTT TTGTCTTGGA ACTCAAGCAC AAGCTTGGTC TTGCTTGAAC   3000
   AAGAAACACT TCTTACCTAC TGCAGAAACC AATCATGTCC TTCGTC                  3046
(2) INFORMATION POUR LA SEQ ID NO: 8:
   (i) CARACTERISTIQUES DE LA SEQUENCE:
      (A) LONGUEUR: 809 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (xi) DESCRIPTION DE LA SEQUENCE: SEQ ID NO: 8:
   ACGTATGTAG TCTTTGTCTA CTTGTAGTTT TTTTTAATTT AAATTAAATA AGTTAATTAG    60
   AGAAATAATA AGAAGGATAT TTTAGTAATT CAACTTTTAA CTTTTAGGTT TCCCACTTAT   120
   AATATAATAT AGATATAGTT TTTTTTAATT TAAATTAAAT AAGTTAATTA GAGAAATAAT   180
   AAGAAGGATA TTTTAGTAAT TCAACTTTTA ACTTTTAGGG TTTCCACTTA TAATATAATA   240
   TAGATATAGA TATAGATATA GATATAGATA AAAGATATAT AGATATAGAT AGATAATATA   300
   GATGGATGAG TCATTGGCGA TAAAGTGAGG ATGTTTCATT TTTGTTATTA AAAACTTACT   360
   ACTCCTTAAA TATAAAATAT GATTCCTTTT AAAAAAGAAA TAGAATAAAA ATAAAGATAA   420
   AACACTAAAA ATAAATTAAT TGTCTAGACA AAATCTACCG TTCACCTCAA TTAATACACA   480
   TCCCCGTCCA CATCATGAAG TAGCTAGCAC AAGCGTACAG ATCAGTTGAA AGAAGAAAAG   540
   GGTCCAGTCC TAAATATCCA AATGTTCATG AAAGGAGGAC AACTTAGTTT TTTCTACTAG   600
   AAAGAATATT TTGACGAATT TCGTTCACAT TGGCATGCTT TAATTTATTA AGTAGTCTTT   660
   CTTGGAAAAG AAGTATTTGC AATATCAAAC CAAATCTTCC CATTACGCAA GCAATGACAT   720
   CTAAGCAAAT ATATATCACT ATAAATAGTA CTACTAATGT TCAATGACTT TTATAAGCAC   780
   TACATATATA TTCTCAAACA AAAAGAATG                                     809
(2) INFORMATION POUR LA SEQ ID NO: 9:
   (i) CARACTERISTIQUES DE LA SEQUENCE:
      (A) LONGUEUR: 331 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (xi) DESCRIPTION DE LA SEQUENCE: SEQ ID NO: 9:
   ACGTATGTAG TCTTTGTCTA CTTGTAGTTT TTTTTAATTT AAATTAAATA AGTTAATTAG    60
   AGAAATAATA AGAAGGATAT TTTAGTAATT CAACTTTTAA CTTTTAGGTT TCCCACTTAT   120
   AATATAATAT AGATATAGTT TTTTTTAATT TAAATTAAAT AAGTTAATTA GAGAAATAAT   180
   AAGAAGGATA TTTTAGTAAT TCAACTTTTA ACTTTTAGGG TTTCCACTTA TAATATAATA   240
   TAGATATAGA TATAGATATA GATATAGATA AAAGATATAT AGATATAGAT AGATAATATA   300
   GATGGATGAG TCATTGGCGA TAAAGTGAGG A                                  331
(2) INFORMATION POUR LA SEQ ID NO: 10:
   (i) CARACTERISTIQUES DE LA SEQUENCE:
      (A) LONGUEUR: 314 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (xi) DESCRIPTION DE LA SEQUENCE: SEQ ID NO: 10:
   TGTTTCATTT TTGTTATTAA AAACTTACTA CTCCTTAAAT ATAAAATATG ATTCCTTTTA    60
   AAAAAGAAAT AGAATAAAAA TAAAGATAAA ACACTAAAAA TAAATTAATT GTCTAGACAA   120
   AATCTACCGT TCACCTCAAT TAATACACAT CCCCGTCCAC ATCATGAAGT AGCTAGCACA   180
   AGCGTACAGA TCAGTTGAAA GAAGAAAAGG GTCCAGTCCT AAATATCCAA ATGTTCATGA   240
   AAGGAGGACA ACTTAGTTTT TTCTACTAGA AAGAATATTT TGACGAATTT CGTTCACATT   300
   GGCATGCTTT AATT                                                     314
(2) INFORMATION POUR LA SEQ ID NO: 11:
   (i) CARACTERISTIQUES DE LA SÉQUENCE:
      (A) LONGUEUR: 161 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (xi) DESCRIPTION DE LA SEQUENCE: SEQ ID NO: 11:
   TATTAAGTAG TCTTTCTTGG AAAAGAAGTA TTTGCAATAT CAAACCAAAT CTTCCCATTA    60
   CGCAAGCAAT GACATCTAAG CAAATATATA TCACTATAAA TAGTACTACT AATGTTCAAT   120
   GACTTTTATA AGCACTACAT ATATATTCTC AAACAAAAAG A                       161
(2) INFORMATION POUR LA SEQ ID NO: 12:
   (1) CARACTERISTIQUES DE LA SEQUENCE:
      (A) LONGUEUR: 307 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (xi) DESCRIPTION DE LA SEQUENCE: SEQ ID NO: 12:
   CTTTTAATAT TCTTCGGCAG AAGAACATTG CTCTTTCCAC GTATGTAGTC TTTGTCTACT    60
   TGTAGTTTTT TTTAATTTAA ATTAAATAAG TTAATTAGAG AAATAATAAG AAGGATATTT   120
   TAGTAATTCA ACTTTTAACT TTTAGGTTTC CCACTTATAA TATAATATAG ATATAGTTTT   180
   TTTTAATTTA AATTAAATAA GTTAATTAGA GAAATAATAA GAAGGATATT TTAGTAATTC   240
   AACTTTTAAC TTTTAGGGTT TCCACTTATA ATATAATATA GATATAGATA TAGATATAGA   300
   TATAGAT                                                             307
(2) INFORMATION POUR LA SEQ ID NO: 13:
   (i) CARACTERISTIQUES DE LA SEQUENCE:
      (A) LONGUEUR: 538 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (xi) DESCRIPTION DE LA SEQUENCE: SEQ ID NO: 13:
   AAAGATATAT AGATATAGAT AGATAATATA GATGGATGAG TCATTGGCGA TAAAGTGAGG 60
   ATTGTTTCAT TTTTGTTATT AAAAACTTAC TACTCCTTAA ATATAAAATA TGATTCCTTT 120
   TAAAAAAGAA ATAGAATAAA AATAAAGATA AAACACTAAA AATAAATTAA TTGTCTAGAC 180
   AAAATCTACC GTTCACCTCA ATTAATACAC ATCCCCGTCC ACATCATGAA GTAGCTAGCA 240
   CAAGCGTACA GATCAGTTGA AAGAAGAAAA GGGTCCAGTC CTAAATATCC AAATGTTCAT 300
   GAAAGGAGGA CAACTTAGTT TTTTCTACTA GAAAGAATAT TTTGACGAAT TTCGTTCACA 360
   TTGGCATGCT TTAATTATAT TAAGTAGTCT TTCTTGGAAA AGAAGTATTT GCAATATCAA 420
   ACCAAATCTT CCCATTACGC AAGCAATGAC ATCTAAGCAA ATATATATCA CTATAAATAG 480
   TACTACTAAT GTTCAATGAC TTTTATAAGC ACTACATATA TATACTCAAA CAAAAAGA 538
(2) INFORMATION POUR LA SEQ ID NO: 14:
   (1) CARACTERISTIQUES DE LA SEQUENCE:
      (A) LONGUEUR: 4284 paires de bases
      (B) TYPE: acide nucléique
      (C) NOMBRE DE BRINS: simple
      (D) CONFIGURATION: linéaire
   (ii) TYPE DE MOLECULE: ADN (génonique)
   (iii) HYPOTHETIQUE: NON
   (xi) DESCRIPTION DE LA SEQUENCE: SEQ ID NO: 14:
   AAGCTTGGCA AGGCTGACCA AAGTCACAGA AGCGATTGGA ATTCGCAGGA CAGACCATGC 60
   ACCTGCGCAC AAAATGTCGT AGGTGCGACA CACCAGAACC AGCACTGGGC AGCAGGTTTC 120
   AATTGCTCTG TGGCTCGTTT GAAACTCATC CGAGCCACTC ATGACCTCGT CCGAATATTT 180
   CAACAAGTCC ATAAACATAA TACGGACATA CTCGGGGTTT CACTTCACGT CAAACAACAT 240
   CAAAATTACA AATCACACCC CGATTCGAAC CTTGAGTTTT AAACTTTTCA ATTTGCAAAT 300
   CTCGTGCCAA AACATATTAA ATGAATCCGG AATGACTTCA AATTTATAAA TGACATAACG 360
   GAGTTGTTCA AATTTCCAGA ATCAGATTCT GCCTTTGATA TCAAAAAGTC AACCCCGTGA 420
   TCAAACTTGG AATTCTTTAG CCTTTAAATT GCTAGTTTTC GTTAAATGGT CATAACTTGA 480
   GCTATGGACC TCCAAATTAA ATTTCGGGCA TACGCTCAAA TCCCAATTAC GAATACGGAG 540
   CTACCGGACT GTCAAAATAC TGATCCGGGT CCGTTTGCTA AAAACGTTGA CCAAAGTCCA 600
   CTAAGTTGAG TTTTAAAACT TTATTTCACA TTTTAATCCA TTTTTTACAT GAAAACTTTC 660
   CGGAAAATAC GGAGTATGCA CGCAAGTCGA GGAATGATAA ATGGTACGTT TCGAAGTTTT 720
   AGAACTCAAA ATTACTTATT AAATTTAAAG ATGACATTTT GGGTCATCAC ATTGATGAAA 780
   ATTTTGACAT TAATATCTGA GAACTTTCTT TGACCTTTTT CGATTCTAAT CCAATCAATT 840
   CAACAGTGTA AGGTGAAGCA GTCAATTTAA AGGAAGGCCT TTAAATTCTA AAATATTGTA 900
   CTTTTCCTGC GCTTCTAAAA GTGAACGACA AAGAAAAAAT AGTTATTCTT GAACTTAATA 960
   TTGTACAATA GGATAAATTT TAACTATCTA TAAAAAGAGA ACAAAACCTT AATCTCTTCA 1020
   AAATAATATT ATAAGAAGTA ACATAATTGT CAAATGAAAT ACACATAAGA AGCACATAAA 1080
   TTTAAATGCC GTATTAAACT TACAGTATAC TATAGCGGAA GTTGGCTTGA TAAAGGAACG 1140
   CTGAGGAGAG TAGCCGATGG TGAAACACTA ACATCAAGTG CAAAAGAAAG AAAAACTGAA 1200
   AACAGAAGAT GAATGTTTGA AGTGGGTAAA AGATTACTTA AAAGATAGGT TTGGTTAACA 1260
   AATGATTGTG ACTGTTACGA AGCAGTGTGA ACCGTTGGGA CTTTTAATAT TCTTCGGCAG 1320
   AAGAACATTG CTCTTTCCAC GTATGTAGTC TTTGTCTACT TGTAGTTTTT TTTAATTTAA 1380
   ATTAAATAAG TTAATTAGAG AAATAATAAG AAGGATATTT TAGTAATTCA ACTTTTAACT 1440
   TTTAGGTTTC CCACTTATAA TATAATATAG ATATAGTTTT TTTTAATTTA AATTAAATAA 1500
   GTTAATTAGA GAAATAATAA GAAGGATATT TTAGTAATTC AACTTTTAAC TTTTAGGGTT 1560
   TCCACTTATA ATATAATATA GATATAGATA TAGATATAGA TATAGATAAA GATATATAGA 1620
   TATAGATAGA TAATATAGAT GGATGAGTCA TTGGCGATAA AGTGAGGATT GTTTCATTTT 1680
   TGTTATTAAA AACTTACTAC TCCTTAAATA TAAAATATGA TTCCTTTTAA AAAAGAAATA 1740
   GAATAAAAAT AAAGATAAAA CACTAAAAAT AAATTAATTG TCTAGACAAA ATCTACCGTT 1800
   CACCTCAATT AATACACATC CCCGTCCACA TCATGAAGTA GCTAGCACAA GCGTACAGAT 1860
   CAGTTGAAAG AAGAAAAGGG TCCAGTCCTA AATATCCAAA TGTTCATGAA AGGAGGACAA 1920
   CTTAGTTTTT TCTACTAGAA AGAATATTTT GACGAATTTC GTTCACATTG GCATGCTTTA 1980
   ATTATATTAA GTAGTCTTTC TTGGAAAAGA AGTATTTGCA ATATCAAACC AAATCTTCCC 2040
   ATTACGCAAG CAATGACATC TAAGCAAATA TATATCACTA TAAATAGTAC TACTAATGTT 2100
   CAATGACTTT TATAAGCACT ACATATATAT ACTCAAACAA AAAGAATGGA GAGCAACAAC 2160
   GTGGTTCTGC TAGATTTCTG GGGGTACGGT CAGTCCCTTA TGTTACGTCC TGTAGAAACC 2220
   CCAACCCGTG AAATCAAAAA ACTCGACGGC CTGTGGGCAT TCAGTCTGGA TCGCGAAAAC 2280
   TGTGGAATTG ATCAGCGTTG GTGGGAAAGC GCGTTACAAG AAAGCCGGGC AATTGCTGTG 2340
   CCAGGCAGTT TTAACGATCA GTTCGCCGAT GCAGATATTC GTAATTATGC GGGCAACGTC 2400
   TGGTATCAGC GCGAAGTCTT TATACCGAAA GGTTGGGCAG GCCAGCGTAT CGTGCTGCGT 2460
   TTCGATGCGG TCACTCATTA CGGCAAAGTG TGGGTCAATA ATCAGGAAGT GATGGAGCAT 2520
   CAGGGCGGCT ATACGCCATT TGAAGCCGAT GTCACGCCGT ATGTTATTGC CGGGAAAAGT 2580
   GTACGTATCA CCGTTTGTGT GAACAACGAA CTGAACTGGC AGACTATCCC GCCGGGAATG 2640
   GTGATTACCG ACGAAAACGG CAAGAAAAAG CAGTCTTACT TCCATGATTT CTTTAACTAT 2700
   GCCGGAATCC ATCGCAGCGT AATGCTCTAC ACCACGCCGA ACACCTGGGT GGACGATATC 2760
   ACCGTGGTGA CGCATGTCGC GCAAGACTGT AACCACGCGT CTGTTGACTG GCAGGTGGTG 2820
   GCCAATGGTG ATGTCAGCGT TGAACTGCGT GATGCGGATC AACAGGTGGT TGCAACTGGA 2880
   CAAGGCACTA GCGGCACTTT GCAAGTGGTG AATCCGCACC TCTGGCAACC GGGTGAAGGT 2940
   TATCTCTATG AACTGTGCGT CACAGCCAAA AGCCAGACAG AGTGTGATAT CTACCCGCTT 3000
   CGCGTCGGCA TCCGGTCAGT GGCAGTGAAG GGCGAACAGT TCCTGATTAA CCACAAACCG 3060
   TTCTACTTTA CTGGCTTTGG TCGTCATGAA GATGCGGACT TACGTGGCAA AGGATTCGAT 3120
   AACGTGCTGA TGGTGCACGA CCACGCATTA ATGGACTGGA TTGGGGCCAA CTCCTACCGT 3180
   ACCTCGCATT ACCCTTACGC TGAAGAGATG CTCGACTGGG CAGATGAACA TGGCATCGTG 3240
   GTGATTGATG AAACTGCTGC TGTCGGCTTT AACCTCTCTT TAGGCATTGG TTTCGAAGCG 3300
   GGCAACAAGC CGAAAGAACT GTACAGCGAA GAGGCAGTCA ACGGCGAAAC TCAGCAAGCG 3360
   CACTTACAGG CGATTAAAGA GCTGATAGCG CGTGACAAAA ACCACCCAAG CGTGGTGATG 3420
   TGGAGTATTG CCAACGAACC GGATACCCGT CCGCAAGTGC ACGGGAATAT TTCGCCACTG 3480
   GCGGAAGCAA CGCGTAAACT CGACCCGACG CGTCCGATCA CCTGCGTCAA TGTAATGTTC 3540
   TGCGACGCTC ACACCGATAC CATCAGCGAT CTCTTTGATG TGCTGTGCCT GAACCGTTAT 3600
   TACGGATGGT ATGTCCAAAG CGGCGATTTG GAAACGGCAG AGAAGGTACT GGAAAAAGAA 3660
   CTTCTGGCCT GGCAGGAGAA ACTGCATCAG CCGATTATCA TCACCGAATA CGGCGTGGAT 3720
   ACGTTAGCCG GGCTGCACTC AATGTACACC GACATGTGGA GTGAAGAGTA TCAGTGTGCA 3780
   TGGCTGGATA TGTATCACCG CGTCTTTGAT CGCGTCAGCG CCGTCGTCGG TGAACAGGTA 3840
   TGGAATTTCG CCGATTTTGC GACCTCGCAA GGCATATTGC GCGTTGGCGG TAACAAGAAA 3900
   GGGATCTTCA CTCGDCGACC GCAAACCGAA GTCGGCGGCT TTTCTGCTGC AAAAACGCTG 3960
   GACTGGCATG AACTTCGGTG AAAAACCGCA GCAGGGAGGC AAACAATGAG AGCTCGAATT 4020
   TCCCCGATCG TTCAAACATT TGGCAATAAA GTTTCTTAAG ATTGAATCCT GTTGCCGGTC 4080
   TTGCGATGAT TATCATATAA TTTCTGTTGA ATTACGTTAA GCATGTAATA ATTAACATGT 4140
   AATGCATGAC GTTATTTATG AGATGGGTTT TTATGATTAG AGTCCCGCAA TTATACATTT 4200
   AATACGCGAT AGAAAACAAA ATATAGCGCG CAAACTAGGA TAAATTATCG CGCGCGGTGT 4260
   CATCTATGTT ACTAGATCGA ATTC 4284
(2) INFORMATION POUR LA SEQ ID NO: 15;
   (i) CARACTERISTIQUES DE LA SEQUENCE:
      (A) LONGUEUR: 58 paires de bases
      (B) TYPE: acide nucléique
      (C) NOMBRE DE BRINS: simple
      (D) CONFIGURATION: linéaire
   (ii) TYPE DE MOLECULE: ADN (génonique)
   (iii) HYPOTHETIQUE: NON
   (xi) DESCRIPTION DE LA SEQUENCE: SEQ ID NO: 15:
   ACTACTAATG TTCAATGACT TTTATAAGCA CTACATATAT ATACTCAAAC AAAAAGAG 58
(2) INFORMATION POUR LA SEQ ID NO: 16:
   (i) CARACTERISTIQUES DE LA SEQUENCE:
      (A) LONGUEUR: 1863 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (xi) DESCRIPTION DE LA SEQUENCE: SEQ ID NO: 16:
   AAGCTTGCAC GACACACTTG TCTACTCCAA AAATATCAAA GATACAGTCC TCAGAAGACC 60
   AAAGGGCCAA TTGAGACTTT TCAACAAAGG GTAATATCCG GAAACCTCCT CGGATTCCAT 120
   TGCCCAGCTA TCTGTCACTT TATTGTGAAG ATAGTGGAAA AGGAAGGTGG CTCCTACAAA 180
   TGCCATCATT GCGATAAAGG AAAGGCCATC GTTGAAGATG CCTCTGCCGA CAGTGGTCCC 240
   AAAGATGGAC CCCCACCCAC GAGGAGCATC GTGGAAAAAG AAGACGTTCC AACCACGTCT 300
   TCAAAGCAAG TGGATTGATG TGATATCTCC ACTGACGTAA GGGATGACGC ACAATCCCAC 360
   TATCCTTCGC AAGACCCTTC CTCTATATAA GGAAGTTCAT TTCATTTGGA GAGAACACGG 420
   GGGACTCTAG AGGATCCATG AGGCGAACTT CTAAATTGAC TACTTTTTCT TTGCTGTTTT 480
   CTCTGGTTTT GCTGAGTGCT GCCTTGGCAC AGAATTGTGG TTCACAGGGC GGAGGCAAAG 540
   TTTGTGCGTC GGGACAATGT TGCAGCAAAT TCGGGTGGTG CGGTAACACT AATGACCATT 600
   GTGGTTCTGG CAATTGTCAA AGTCAGTGTC CAGGTGGCGG CCCTGGTCCT GGTCCTGTTA 660
   CTGGTGGGGA CCTCGGAAGC GTCATCTCAA ATTCTATGTT TGATCAAATG CTTAAGCATC 720
   GTAACGAAAA TTCTTGTCAA GGAAAGAATA ATTTCTACAG TTACAATGCC TTTATTACTG 780
   CTGCTAGGTC TTTTCCTGGC TTTGGTACAA GTGGTGATAT CAATGCCCGT AAAAGGGAAA 840
   TTGCTGCTTT CTTTGCCCAA ACCTCCCATG AAACTACTGG TATGTGTATA ACCATTCACA 900
   TCGAACCATT AAAATATAAT TTCATTTTAT TTTATTTAGT AATTGATTAT ATATGTAGGA 960
   GGATGGCCTT CCGCACCTGA TGGACCATTC GCATGGGGTT ACTGTTTCCT TAGAGAACGA 1020
   GGTAACCCCG GTGACTACTG TTCACCAAGT AGTCAATGGC CTTGTGCACC TGGAAGGAAA 1080
   TATTTCGGAC GAGGCCCAAT CCAAATTTCA CAGTAAGCTA CATAAATCTA TATATGGTAA 1140
   AATTTGATGA ACTTGTAGTG TCTAATTACG TGTATTTTGA CATTTCAAAA CAGCAACTAC 1200
   AACTATGGGC CATGTGGAAG AGCCATCGGA GTGGACCTTT TAAACAATCC TGATTTAGTA 1260
   GCCACAGACC CAGTCATCTC ATTCAAGACT GCTATCTGGT TCTGGATGAC CCCTCAATCA 1320
   CCAAAGCCTT CTTGCCACGA TGTCATCATT GGAAGATGGA ACCCATCTGC CGGTGACCGA 1380
   TCAGCCAATC GTCTTCCTGG ATTTGGTGTC ATCACAAACA TCATCAATGG GGGCCTGGAA 1440
   TGTGGTCGTG GCAATGACAA TAGGGTCCAG GATCGCATTG GGTTTTACAG GAGGTATTGC 1500
   GGTATTCTTG GTGTTAGTCC TGGTGACAAT CTTGATTGCG GAAACCAGAG ATCTTTTGGA 1560
   AACGGACTTT TAGTCGATAC TATGTAATGA GCTCGAATTT CCCCGATCGT TCAAACATTT 1620
   GGCAATAAAG TTTCTTAAGA TTGAATCCTG TTGCCGGTCT TGCGATGATT ATCATATAAT 1680
   TTCTGTTGAA TTACGTTAAG CATGTAATAA TTAACATGTA ATGCATGACG TTATTTATGA 1740
   GATGGGTTTT TATGATTAGA GTCCCGCAAT TATACATTTA ATACGCGATA GAAAACAAAA 1800
   TATAGCGCGC AAACTAGGAT AAATTATCGC GCGCGGTGTC ATCTATGTTA CTAGATCGAA 1860
   TTC 1863
(2) INFORMATION POUR LA SEQ ID NO: 17:
   (i) CARACTERISTIQUES DE LA SEQUENCE:
      (A) LONGUEUR: 18 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (xi) DESCRIPTION DE LA SEQUENCE: SEQ ID NO: 17:
   GAANNGAANN TTCNNTTC 18
(2) INFORMATION POUR LA SEQ ID NO: 18:
   (i) CARACTERISTIQUES DE LA SEQUENCE:
      (A) LONGUEUR: 18 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (xi) DESCRIPTION DE LA SEQUENCE: SEQ ID NO: 18:
   TTCNNTTCNN GAANNGAA 18

## Description

La présente invention a pour objet un nouveau promoteur végétal et les microorganismes et cellules végétales contenant une unité d'expression d'une protéine d'intérêt comprenant ledit promoteur. Le promoteur selon l'invention est un promoteur constitutif fort permettant une expression de ladite protéine dans les microorganismes et les cellules végétales quelque soit le stade de développement du végétal.

De plus, le promoteur selon l'invention trouve une application particulière dans le domaine de la protection des végétaux par génie génétique et notamment celui de la défense des plantes en état de stress.

Au cours des dernières années, les applications de la transformation des végétaux se sont multipliées. De nombreux gènes d'origine procaryote ou eucaryote (animale ou végétale) codant notamment pour des protéines conférant lors de leur expression un caractère agronomique nouveau, ont été isolés puis transférés aux plantes.

Dans de très nombreux cas, les gènes qui ont été introduits par génie génétique dans les plantes, sont chimériques, associant des éléments de régulation de différentes origines. C'est ainsi que très souvent le gène codant pour une protéine d'intérêt est placé sous le contrôle d'un promoteur constitutif fort permettant une expression de ladite protéine dans toute la plante (ou la majeure partie de celle-ci) durant toute sa vie, quel que soit le stade de développement. Le promoteur du transcrit 35S du virus de la mosaïque du choux-fleur (35S CaMV), le plus utilisé dans les constructions de gènes chimériques, correspond à cette description.

Or pour un certain nombre d'applications, il n'est pas nécessaire que le gène codant pour la protéine d'intérêt, support du caractère agronomique, ait une expression continue ou répartie dans toute la plante. De telles caractéristiques peuvent même dans certains cas amoindrir ou annuler les effets bénéfiques du gène transféré. En effet, l'expression continue à un niveau élevé d'une protéine peut détourner une partie du métabolisme vers cette expression, et finalement entraîner une perte de rendement.

Très tôt, la recherche d'une expression génique plus spécifique a été engagée ; elle a conduit par exemple à isoler des promoteurs tissus- ou organes-spécifiques.

Il peut être intéressant d'induire l'expression d'un gène donné uniquement dans une situation précise, ou mieux d'assurer un niveau d'expression de base tout au long de la vie d'un végétal, tout en permettant la surexpression du gène dans une situation donnée.

Plusieurs promoteurs inductibles ont déjà été décrits, certains répondant à la fois à l'infection par des microorganismes pathogènes et à des composés chimiques ou des hormones végétales comme l'éthylène (Roby et al, 1990, Plant Cell 2, 999) ou l'auxine.

Un promoteur végétal isolé du tabac a été décrit par Takahashi et al., Proc. Natl. Acad. Sci. USA, 1990, 87, 8013-8016.

Ces auteurs, dans une autre publication précisent que ce promoteur ne réagit spécifiquement qu'aux auxines et non aux autres hormones ou aux stress (Takahashi et al., The Plant Journal, 1991, 1(3), 327-332).

La présente invention a pour objet un promoteur, qui s'exprime à un niveau soutenu dans les différents organes et tissus d'un végétal et notamment les racines et le méristème d'une plante et qui est très fortement inductible dans les situations de stress, telles que notamment après un choc thermique, une blessure, un choc hormonal, un éliciteur biotique ou abiotique ou une infection bactérienne, fongique ou virale.

L'invention concerne également les cellules végétales à l'exception de cellules végétales présentes à l'état naturel, les microorganismes (bactéries) et les cellules de microorganismes ayant intégré une unité d'expression d'une protéine d'intérêt, ladite unité comprenant le promoteur selon l'invention.

Elle a également pour objet les végétaux ou parties de végétaux ainsi que les semences qui comprennent les cellules végétales de l'invention.

Enfin, elle concerne aussi l'utilisation d'un végétal ou d'une partie d'un végétal ci-dessus pour sélectionner des molécules à activité phytosanitaire susceptibles d'induire des réactions de défense naturelles des végétaux contre les agressions d'organismes phytopathogènes ou phytophages (champignons, bactéries, virus, insectes et nématodes).

Le promoteur selon l'invention comprend la séquence d'ADN (B) [SEQ ID NO : 4] ou une séquence présentant un degré d'homologie d'au moins 80% avec la séquence (B).

Selon une variante, le promoteur selon l'invention comporte, en amont de la séquence (B), une séquence (C) [SEQ ID NO : 5] ou une séquence présentant un degré d'homologie élevé avec la séquence (C).

Enfin, selon une variante préférée de l'invention, le promoteur de l'invention comporte, en amont de la séquence (B), une séquence (D) [SEQ ID NO : 6] ou une séquence présentant un degré d'homologie élevé avec la séquence (D).

Un degré d'homologie élevé. signifie ici une homologie (rapport entre les nucléotides identiques et le nombre total de nucléotides) d'au moins 70 %. et de préférence d'au moins 80 %, des séquences de nucléotides, lorsqu'elles sont alignées d'après l'homologie maximale, selon la méthode d'alignement optimal des séquences de Needleman et Wunsch. 1970; J. Mol. Biol., 48, 443-453. Cette méthode est notamment utilisée dans le logiciel UWGCG de l'Université du Wisconsin : Devereux et al., 1984, Nucl. Ac. Res., 12, 387-395 - option GAP.

Les éléments nécessaires au fonctionnement de ce promoteur et à l'expression de ses caractéristiques (facteurs transactivateurs, etc) sont présents dans d'autres végétaux, dicotylédones ou monocotylédones. Leur présence permet donc l'utilisation de ce promoteur dans de nombreux végétaux cultivés, tels que notamment les plantes (tabac, pomme de terre, tomate, maïs, tournesol, orge et colza) ou d'autres végétaux, tels que les levures et les champignons.

Toutes séquences d'ADN codant pour des protéines d'intérêt peuvent être placées sous le contrôle du promoteur selon l'invention, en particulier des séquences codant pour des protéines qui permettent d'assurer la protection d'un végétal, par exemple une plante contre les infections virales, bactériennes ou fongiques et contre les autres états de stress. A titre d'exemples de telles protéines, on peut citer notamment les endochitinases de tomate-tabac, telles que celle décrite dans EP-A-493 581 dont la séquence codante est [SEQ ID NO : 16].

Le promoteur selon l'invention a été obtenu par criblage d'une banque génomique de tabac à l'aide d'une sonde d'ADN ayant la séquence [SEQ ID NO : 1].

Un clone correspondant à la séquence [SEQ ID NO : 1 ] a été obtenu par screening différentiel de clones d'ADNc issus d'ARNm poly(A)⁺, spécifiquement synthétisés au cours de l'infection de feuilles de tabac *Nicotiana tabacum* par la souche de *Pseudoaonas solanacearum* GMI 1000. Cette souche bactérienne est bien connue pour provoquer une réaction d'hypersensibilité sur le tabac de la variété *Bottom spécial.* A cet effet, on peut se référer à l'ouvrage Message et al, 1978. Proc. 4th. Intl. Conf. of plant Pathogenic Bacteria pp. 823-833. Le clone contenant la séquence [SEQ ID NO : 1] sera dénommé ci-après "clone 246".

Le clone 246 a permis ensuite, par criblage d'une banque génomique de tabac d'isoler un clone, dénommé ci-après clone 246 C [SEQ ID NO : 7]. qui contient la séquence d'ADN (D) [SEQ ID NO : 6], la séquence d'ADN (C) [SEQ ID NO : 5 ], la séquence d'ADN (B) [SEQ ID NO : 4 ] et une séquence renfermant 2 cadres de lectures ouverts séparés par un intron.

Par association du promoteur (séquences B+C+D) avec le gène de la β-glucuronidase on a obtenu, selon le mode opératoire décrit a la section 9 ci-après, le vecteur d'expression pSG 123. Le promoteur constitué des séquences B+C+D est appelé promoteur 246C.

Le vecteur d'expression pSG 123 a été utilisé pour tester l'expression transitoire dans des protoplastes de tabac, du gène de la glucuronidase, lesdits protoplastes étant mis en situation de stress soit par infection par *Pseudomonas solanacearum* soit par traitement à l'aide d'un éliciteur ou d'une hormone.

A partir du vecteur d'expression pSG 123 on a préparé, selon le mode opératoire décrit à la section 13, un vecteur d'expression stable dans les cellules végétales, le vecteur binaire pSG 246.

Ce vecteur binaire pSG 246 a été transféré dans des cellules *d'Agrobacterium tumefaciens* ou *Agrobacterium rhizogenes,* lesquelles ont été utilisées ensuite pour obtenir des plantes transgéniques de tabac, de colza et de tournesol. Pour la transformation des tissus de monocotylédones (orge et maïs) le vecteur d'expression pSG 123 a été utilisé. On a étudié le comportement de ces plantes ou tissus en état de stress.

Le promoteur selon l'invention, comprenant la séquence (B), (C) et (D) a aussi été associé au gène codant pour la chitinase tomate-tabac. Les gènes chimériques résultants ont été utilisés pour transformer des cellules *d'Agrobacterium.*

La séquence d'ADN [SEQ ID NO : 1] peut aisément être synthétisée selon les techniques bien connues de l'homme de métier (L.J. Mac PRIDE et H.M.CARUTHURS Tetrahydron letters (1983) vol. 24 : 245).

L'étude des plantes transgéniques obtenues par transformation de plantes à l'aide de cellules d'*Agrobacterium* obtenues ci-dessus a permis de mettre en évidence l'activité promotrice de base du promoteur selon l'invention ainsi que la surexpression des protéines d'intérêt (β-glucuronidase et chitinase) dans des situations de stress.

Les résultats figurant dans la partie illustrative ci-après montrent clairement que le promoteur selon l'invention a une activité promotrice de base qui est fortement augmentée lorsque les plantes transgéniques contenant ce promoteur et un gène codant pour une protéine d'intérêt, sont placées dans des conditions de stress : choc thermique, blessure, infection par des pathogènes (champignons, bactéries), éliciteurs (biotiques et abiotiques).

Par différentes délétions du plasmide pSG 123, effectuées dans la région 5' du promoteur à l'aide d'enzymes de restriction et/ou de la nucléase Exo3 on a mis en évidence les vecteurs pSG 251 et pSG 33 dont les séquences sont respectivement la séquence (B) (pSG 33) [SEQ ID NO : 4 ] ou la séquence (B) comportant en amont la séquence (C) (pSG 251) [SEQ ID NO : 5].

La visualisation aisée de l'expression de la glucuronidase (Jefferson et al., 1987, Plant Molec. Biol. Reporter, 5. 387) ou de sa surexpression dans le cas d'une induction du promoteur de l'invention, permet d'utiliser des plantes qui expriment ce gène chimérique pour la sélection de molécules inductrices.

Les plantes possèdent des mécanismes de défense aux agressions et notamment aux agressions parasitaires (champignons, bactéries, virus ou insectes) ; ces mécanismes dépendant de phénomènes d'induction sont peu connus et se mettent souvent en place trop tardivement pour être efficaces. Leur déclenchement précoce (Roby et al.. 1988, Physiol. Molec. Plant. Pathol., 33. 409) notamment par des composés de type éliciteur dans des réactions en cascade permet à la plante de résister aux agressions.

Des fongicides de seconde génération, actifs sur les défenses de la plante, tout en étant inactifs sur le parasite ont déjà été mis sur le marché.

Les plantes exprimant la glucuronidase, sous contrôle du promoteur de l'invention, précocement et spécifiquement induit lors d'une réaction d'hypersensibilité à une infection bactérienne, constituent un outil de choix pour sélectionner des molécules capables d'induire l'expression ou la surexpression de gène de défense.

L'invention sera mieux comprise à l'aide de l'exposé ci-après, divisé en sections, qui comprend des résultats expérimentaux et une discussion de ceux-ci. Certaines de ces sections concernent des expériences effectuées dans le but de réaliser l'invention, d'autres des exemples de réalisation de l'invention, donnés bien sûr à titre purement illustratif.

Une grande partie de l'ensemble des techniques ci-après, bien connues de l'homme du métier, est exposée en détail dans l'ouvrage de Sambrook et al. : "Molecular Cloning : a Laboratory manual" publié en 1989 par les éditions Cold Spring Harbor Press à New-York (2ème édition).

Le matériel biologique (souches, phages, plasmides ou plantes) utilisé dans les sections ci-après est disponible dans le commerce et/ou décrit respectivement dans les documents ci-après :

| | |
|---|---|
| - vecteur binaire pBIN 19 | BEVAN et al., 1984, Nucl. Ac. Res., 12. 8711-8721 ; obtenu auprès de Clontech (Palo Alto Californie USA) |
| - vecteur 101.3 | JEFFERSON, 1987, Plant. Molec. Biol.Reporter 5, 387 - 405 obtenu auprès de Clontech |
| - vecteur pBI 221 | " |
| - vecteur pBI 121 | " |
| - Souche *Pseudomonas solanacearum* | MESSAGE et al., 1978, Proc. 4th Intl. Conf.of Plant Pathogenic Bacteria pp 823-833 |
| - Souche *Pseudomonas solanacearum* | " |
| GMI 1178 | " |
| - Souche *Pseudomonas solanacearum* | " |
| K 60 | " |
| - terminateur NOS | terminateur nopaline synthase |
| - vecteur pTZ 19R | obtenu auprès de Pharmacia |
| - souche *E. coli* MC1061 | MANIATIS et al., 1982, Molecular cloning : A laboratory Manual, Cold Spring Harbor, New-York, obtenue auprès Clontech |
| - souche *E. coli* HB101 | " |
| - souche *Agrobacterium tumefaciens* | LBA4404 obtenue auprès de Clontech HOEKEMA et al.. 1983, NATURE. 303, 179-180 ; |
| - souche *Agrobacterium rhizogenes* | pRIA₄ |
| - plante *Nicotiana tabacum* | Variété Wisconsin Havana 38 : SCHNEIDER M., 1990, Plant Molec. Biol., 14, 935-947 ; |
| - champignon *Chalara elegans* | RAWLINGS R.E, 1940. Ann. Mo. Bot. Gdn., 27, 561-598 ; |
| - champignon *Alternaria brassicae* | BAINS et TEWARI, 1987, Physiol. Mol. Plant. Pathol. 30 : 259 ; |
| - plante *Nicotiana tabacum* | Variété Paraguay 49 obtenue auprès de l'institut du tabac, Bergerac, France. |
| - plante *Brassica napus* | variétés de printemps Brutor et Westar et lignée d'hiver (lignée de sélection Rustica Semences) |
| - pathogène *Rhzizoctonia Solani* | ACHARYA et al., 1984, Can. J. Plant. Pathol, 6, 325-328 |
| - plantes de tournesol | genotype 2603B (lignée de sélection, Rustica semences) |
| - maïs | lignée LH 132 |
| - orge | variété GERBEL obtenue auprès de l'Institut National de la Recherche Agronomique (INRA), Paris, France |

- Les souches GMI 1000 et K 60 peuvent être obtenues auprès de la Collection Nationale des Bactéries Phytopathogènes (CNBP) INRA. Pathologie Végétale. Rue Georges MOREL, 49070 BEAUCOUZE, FRANCE
- La souche GMI 1178 peut être obtenue auprès de l'INRA, Pathologie Végétale, Chemin de Borde-Rouge AUZEVILLE BP 27, 31326 CASTANET TOLOSAN Cedex, FRANCE

Les abréviations suivantes sont utilisées dans les exemples ci-après :
32P-dCTP: déoxycytidine 5'-³²P-triphosphate commercialisé par AMERSHAM sous la référence 10205 ;
2 SSC : NaCl 0,3M, citrate trisodique 30 mM ; pH 7,0 (décrit par MANIATIS et al., op. cit.) ;
SDS : dodécylsulfate de sodium ;
FPLC : chromatographie liquide rapide de protéines
PVDF : difluorure de polyvinylidène ;
EDTA : acide éthylènediaminetétraacétique
DEPC : diéthylpyrocarbonate.
NAD : nicotinamide adénine dinucléotide

La description ci-après sera mieux comprise en se rapportant aux figures 1 à 3.

La figure 1 représente la cartographie du clone d'ADN génomique 246C, établie à l'aide des enzymes de restriction représentés.

La figure 2 représente l'alignement selon la méthode d'alignement optimal de Needleman et Wunsch, 1970, J. Mol., BIol., 48, 443-453 mise en oeuvre par le logiciel UWGCG de l'Université du Wisconsin (Devereux et al., 1984, Nucl. Ac. Res., 12, 387-395) option GAP, de la partie 3' de 700 pb du promoteur du gène 246C et du promoteur décrit par Takahashi et al., 1989,Proc. Natl. Acad. Sci. USA, 87, 8013).

La figure 3 représente les différents vecteurs d'expression testés comportant, par rapport au plasmide pleine longueur pSG123, une délétion variable de la partie 5' du promoteur.

### Section 1 : Infection de tabac Nicotiana tabacum par deux souches de la bactérie pathogène Pseudomonas solanacearum

Les bactéries de la souche de *Pseudomonas solanacearum* sont cultivées pendant 72 h sur le milieu BG gélosé (Boucher et al. 1985. J. Gen Microbiol 131, 2449) ; une colonie est prélevée pour ensemencer 40 ml du même milieu. Après de 16 à 24 h d'incubation à 28°C. la suspension bactérienne est centrifugée pendant 10 min à 6000 g et 4°C ; le surnageant est éliminé en conservant la couche de polysaccharides présente à la surface du culot. Après un lavage à l'eau stérile, les bactéries sont remises en suspension dans 20 ml d'eau. Leur concentration est alors déterminée par densitométrie.

Des jeunes feuilles de plantes de tabac sont détachées de la plante. lavées à l'eau distillée et immergées dans un dessicateur contenant 1,2 l de suspension bactérienne à la concentration de 3 x 10⁶ bactéries/ml. Le vide est réalisé pendant 10 min à l'aide d'une trompe à vide, puis il est lentement cassé. Chaque feuille infiltrée est alors placée dans un bêcher contenant du tampon phosphate de sodium 10 mM pH 6,0 et transférée dans une chambre de culture. Les feuilles sont prélevées après différents temps d'incubation et conservées à - 70°C.

Deux souches bactériennes ont été utilisées : la souche GMI 1000 provoquant une réaction d'hypersensibilité sur le tabac de la variété Bottom Spécial et la souche GMI 1178 (Message et al, 1978, Proc. 4th. Intl. Conf. of Plant Pathogenic Bacteria pp. 823-833), mutant issu de la souche précédente et ayant perdu la capacité d'induire la réaction d'hypersensibilité chez le tabac.

### Section 2 : Extraction et isolement des ARN totaux de Nicotiana tabacum infectés par les souches de Pseudomonas solanacearum

10 g de matériel végétal sont broyés en présence d'azote liquide puis repris dans un mélange de 3 ml de phénol, 3 ml de chloroforme-alcool isoamylique (24 : 1, V : V) et 6 ml de tampon de lyse de composition Tris-HCl 200 mM pH 7,0. EDTA 200 mM, SDS 1 %.

Après agitation au vortex, une centrifugation pendant 10 min à 6000 g à 4°C permet de séparer les phases. La phase aqueuse est prélevée et reextraite à l'aide de 6 ml de mélange de phénol-chloroforme-alcool isoamylique (25 : 24 : 1, V : V). puis à l'aide de 6 al de phénol. 16 ml d'éthanol absolu et 400 µl d'acétate de sodium 3M pH 5.5 sont ajoutés à la phase aqueuse ; le mélange est précipité pendant 2 h à - 20°C. Le culot obtenu est dissous dans 5 ml d'eau stérile contenant 0.1 % de diethylpyrocarbonate (DEPC) puis les ARN sont précipités pendant 12 h à 4°C après addition de 5 ml de LiCl 4M. Après une centrifugation de 20 min à 6000 g, le culot d'ARN est lavé par de l'éthanol à 75 %. séché et repris dans 800 µl d'eau distillée stérile contenant 0.1 % de DEPC. La solution est précipitée pendant 2 h à - 20°C après addition de 0.1 vol d'acétate de sodium 3M pH 5,5 et 2,5 vol d'éthanol absolu. Après centrifugation pendant 15 min à 12 000 g, le culot d'ARN est lavé à l'éthanol à 75 % et dissous dans 200 µl d'eau distillée contenant 0,1 % de DEPC.
Les ARN totaux sont alors dosés par spectrophotométrie à 260 nm.

### Section 3 : Préparation des ARN messagers poly(A)⁺

1 g de gel d'oligo-d(T) cellulose (Collaborative Research Inc.) est remis en suspension dans 2 ml de tampon Tris-HCl 10 mM pH 7,4, EDTA 1 mM, NaCl 0,4 M, SDS 0,1 %, Ce gel est introduit dans une pipette Pasteur dont l'extrémité est bouchée à l'aide de laine de verre. La solution d'ARN est portée à 65°C pendant 4 min puis laissée refroidir lentement à la température ambiante. Une concentration finale de 0.4 M en NaCl est obtenue par addition d'une solution de NaCl 5M.

La solution d'ARN totaux est ensuite déposée sur le gel d'oligo-d(T) cellulose. Celui-ci est ensuite rincé par 12 ml environ de tampon de composition Tris-HCl 10 mM pH 7.4, NaCl 0,4 M, EDTA 1 mM, SDS 0,5 %.

Les ARN poly(A)⁺ sont ensuite élues à l'aide de 7 ml de tampon de composition Tris-HCl 10 mM pH 7,4, SDS 0.1 X, puis précipités pendant 1 nuit à -20°C après addition de 2,5 volumes d'éthanol à 95 % et 0,1 volume d'acétate de sodium 3,3 M pH 5,5. Le culot d'ARN poly(A)⁺, obtenu par centrifugation à 35 000 g pendant 1 h est lavé 3 fois par de l'éthanol à 75 %, séché, repris dans 0,5 ml d'eau distillée stérile et soumis à une nouvelle précipitation dans les conditions décrites ci-dessus. Après centrifugation, le culot est alors lavé par de l'éthanol à 75 %, séché et dissous dans de l'eau distillée stérile. Les ARN poly(A)⁺ sont alors dosés par spectrophotométrie à 260 nm.

### Section 4 : Synthèse de l'ADN double brin à partir d'ARN messagers poly(A)⁺ isolés de feuilles de tabac infecté par la souche GMI 1000 de Pseudomonas solanacearum et clonage dans E. coli.

Cette synthèse est réalisée selon la méthode de Gubler et Hoffman (1983, Gene, 25, 263), à l'aide du kit D. Scribe de la société Genofit (Genève, Suisse) et en suivant les instructions du fabricant.

### Synthèse du premier brin :

2,5 µg d'ARN poly(A)⁺ isolés de feuilles de tabac infectées par la souche GMI 1000 de *Pseudomonas solanacearum* et prélevées 6 h après inoculation, sont mis à incuber à 44°C pendant de 30 à 60 min en présence de : Tris HCl 40 mM, pH 8,3, NaCl 80 mM, MgCl₂ 6 mM, DTT 5 mM, dATP 0,5 mM, dTTP 0,5 mM, dGTP 0,5 mM, dCTP 0,5 mM, 1,5 µg d'oligo d (pT) 12-18 et 10 à 15 unités de transcriptase inverse d'AMV (avion myeloblastosis virus) dans un volume total de 25 µl.

### Synthèse du deuxième brin :

Le milieu réactionnel issu de la synthèse du premier brin est diluée à 100 µl à l'aide du tampon de composition Tris HCl 40 mM pH 7,5, MgCl₂ 10 mM, NaCl 80 mM ; 2 unités de RNase H sont ajoutées et le mélange est incubé à 37°C pendant 5 min.

Après refroidissement à 12°C, 25 unités de DNA polymérase I, 0,5 unités Weiss de ligase de *E. coli* et du NAD en concentration finale de 0,1 mM, sont ajoutés.

Après une incubation de 60 min à 12°C puis de 60 min à 18°C, la réaction est arrêtée par l'addition de EDTA et de SDS en concentrations finales de 20 mM et 1 %, respectivement, suivie d'un chauffage de 2 min à 60°C.

### Purification de l'ADNc :

Celle-ci est réalisée par chromatographie sur une colonne de Sephadex G100, dans le tampon Tris HCl 50 mM pH 7,5, NaCl 300 mM, EDTA 1mM. L'addition d'alpha 32P dCTP lors de l'étape de synthèse permet de repérer plus facilement les fractions issues de la chromatographie renfermant l'ADN double brin. Celui-ci est précipité par addition d'acétate d'ammonium (concentration finale 0,3 M) et de 2,5 vol d'éthanol absolu.

### Digestion à l'aide de la nucléase S1 :

Le précipité obtenu est lavé par de l'éthanol à 75 %, séché et repris par de l'eau stérile. L'ADNc est traité ensuite par la nucléase S1 dans un volume total de 300 µl, à 30°C pendant 10 min, en présence de 20 U d'enzyme dans le tampon de composition acétate de sodium 30 mM pH 4,4, NaCl 0,25 M, ZnCl₂ 1 mM.

A la fin de la réaction, le mélange est extrait une fois par un mélange de Phénol-chloroforme-alcool isoamylique (50-48-2. V : V) puis trois fois par l'éther éthylique avant d'être soumis à une précipitation par l'éthanol.

Le précipité d'ADN obtenu après centrifugation est ensuite séché et repris dans 20 µl d'eau stérile.

### Addition en 3' d'une terminaison Poly dC (dC Tailing)

A la solution d'ADNc sont ajoutés successivement 10 µl de tampon de terminal-transférase de composition potassium cacodylate 70 mM pH 7,2, CoCl₂ 0,5 mM, dithiothréitol 0,5 mM, 2 µl d'α dCTP 0,25 mM, 5 µl d'α 32P dCTP (370 MBq/ml), 12 µl d'H₂O et 1,2 µl de terminal transférase (18 unités).

La réaction de tailing est réalisée par incubation à 37°C pendant 30 min, puis arrêtée par l'addition de 30 µl d'EDTA, 0,25M pH 8.0.

### Purification après Tailing des ADNc double brin :

Le mélange issu du dC tailing est précipité par l'éthanol puis centrifugé. Le culot d'ADNc est repris dans 10 µl de tampon de composition Tris HCl 10 mM pH 7,5, NaCl 100 mM, EDTA 1 mM, 5 % glycérol, 0,02 % bleu de bromophénol. Cette solution est déposée sur une colonne de Biogel A 0,5 M (1 ml de support). L'élution de la colonne est réalisée par le tampon de composition Tris HCl 10 mM, pH 7,5, NaCl 100 mM, EDTA 1 mM ; des fractions sont recueillies, leur radioactivité mesurée, et la taille des ADNc qu'elles renferment est analysée en électrophorèse sur gel d'agarose. Les fractions contenant des ADNc de taille supérieure à 500 paires de bases sont réunies et l'ADNc est précipité par l'éthanol.

### Hybridation avec pBR 322-dG

L'ADNc recueilli après précipitation est remis en solution dans 50 µl de tampon de circularisation de composition Tris-HCl 20 mM pH 7.5. NaCl 100 mM, EDTA 1mM. 4 µl de solution de pBR 322-dG (21 ng, Clontech) et 46 µl d'eau sont rajoutés.

Après incubation pendant 15 min à 65°C puis 2 h à 57°C. l'hybridation est contrôlée par électrophorèse sur gel d'agarose.

L'ADNc double brin obtenu est inséré au site PstI du plasmide pBR 322. Après ligation, l'ADN obtenu est utilisé pour transformer des cellules compétentes de la souche *E. coli* HB101 Des colonies de bactéries recombinantes sont obtenues après étalement et culture sur boite de Pétri des cellules compétentes transformées.

### Section 5 : Sélection par screening différentiel de clones d'ADNc issus d'ARNm poly(A)⁺ spécifiquement synthétisés au cours de l'infection bactérienne par la souche de Pseudomonas solanacearum GMI 1000.

L'ADN des colonies bactériennes recombinantes obtenues par clonage d'ADNc synthétisé à partir d'ARN messagers poly(A)⁺ de feuilles de tabac infectées par la souche GMI 1000 a été transféré sur une membrane de nylon Biodyne (Pall Corporation, EUA) suivant les indications du fabricant. Ces ADN sont hybridés successivement à l'aide de 2 sondes radioactives obtenues par synthèse d'ADNc, en présence d'alpha-32P dCTP, à partir d'ARN messagers purifiés de plantes infectées par les souches GMI 1000 et GMI 1178 respectivement.

Après lavage et exposition autoradiographique des membranes, les colonies bactériennes qui présentent un signal d'hybridation plus fort avec la sonde synthétisée à partir de feuilles inoculées par la souche GMI 1000 sont sélectionnées. L'ADN plasmidique de ces colonies est préparé, les inserts d'ADNc de ces plasmides sont isolés puis marqués à l'alpha 32-dCTP et sont utilisés comme sonde pour révéler selon la technique de dot-blot puis de Northern blot les quantités équivalentes d'ARNm correspondant purifié à partir d'ARN total, de feuilles de tabac inoculées par la souche GMI 1000 ou GMI 1178.

Les colonies dont l'insert utilisé comme sonde donne un signal plus intense lors de la révélation des ARN totaux purifiés à partir de feuilles de tabac inoculées par la souche GMI 1000 sont conservées.

### Section 6 : Caractérisation d'un clone d'ADNc issu d'un ARNm spécifiquement synthétisé au cours de l'infection bactérienne par la souche de Pseudomonas solanacearum GMI 1000.

14 clones bactériens ont été isolés à partir de la banque d'ADNc construite à partir d'ARN messagers de feuilles de tabac infectées par la souche de *Pseudomonas solanacearum* GMI 1000.

Parmi ceux-ci, un clone appelé 246. présentant une longueur d'insert de 750 paires de bases, permet de révéler en Northern Blot un transcrit de 800 nucléotides environ. L'accumulation de ce transcrit commence 4 à 9 h après l'inoculation, et atteint un maximum entre 12 et 15 h. Dans les feuilles de tabac infectées par la souche GMI 1178, on observe une légère accumulation du transcrit entre 12 et 15 h.

L'étude de la séquence de l'ADNc du clone 246 [SEQ ID NO : 1] met en évidence l'existence d'un premier cadre de lecture ouvert incomplet. codant pour un peptide de 59 acides aminés et d'un second cadre potentiel codant pour un peptide de 88 acides aminés. La séquence de ces deux peptides est représentée par [SEQ ID NO : 2]. Entre ces deux cadres de lecture des séquences consensus d'épissage d'un intron (Brown, 1986, Nucl. Ac. Res., 14, 9549) sont présentes. Il y a donc probablement eu clonage d'un ADNc à partir d'un ARN messager immature. La séquence d'ADNc du clone 246 sans l'intron est la séquence A₁ [SEQ ID NO : 3].

### Section 7 : Criblage d'une banque génomique de tabac à l'aide de l'ADNc caractérisé.

Une banque génomique d'ADN de tabac a été obtenue par digestion partielle à l'aide de l'enzyme MboI d'ADN isolé de germination de *Nicotiana tabacum* variété NK 326, et clonage des fragments de restriction dans le phage EMBL-3 (Clontech). 500 000 phages recombinants ont été criblés, après étalement à raison de 10 000 phages par boîte de Petri selon la technique connue de l'homme de l'art et décrite dans Sambrook et al (Molecular Cloning, A laboratory manual, Cold Spring Harbor Laboratory Press, 1989).

L'ADN phagique est transféré sur membrane de nitrocellulose (BA 85 Schleicher et Schüll), dénaturé pendant 2 min dans une solution NaOH 0,5 M, NaCl 1,5 M, puis trempé dans une solution de neutralisation NaCl 1,5 M, Tris HCl 0,5 M pH 7,4 pendant 5 min. Après rinçage rapide dans du 2 SSC (NaCl 0,3 M, citrate de sodium 30 mM) les filtres sont séchés 30 min à 37°C et l'ADN est ensuite fixé par traitement à 80°C sous vide pendant 1h30.

Les membranes sont ensuite préhybridées pendant 4 h à 37°C dans un tampon de composition 5 SSC (NaCl 0,75 M, citrate de sodium 75 mM), 50 % formamide, 0,3 % lait écrémé.

L'hybridation est réalisée dans le même tampon, pendant 18 h à 37°C après addition de la sonde marquée à l'alpha 32P-dCTP, constituée de l'insert de 750 paires de bases du clone d'ADNc 246.

Les membranes sont ensuite lavées 3 fois pendant 20 min à 37°C dans une solution 5 SSC, SDS 0,1 %, puis 2 fois pendant 30 min à 37°C dans une solution 2 SSC, SDS 0,1 % et enfin 30 min à 42°C dans une solution 2 SSC, SDS 0,1 % ; elles sont ensuite autoradiographiées.

Après révélation des autoradiogrammes, chaque plage de lyse présentant un signal positif est isolée, les phages sont élués dans du milieu SM (NaCl 100 mM, Tris HCl 50 mM, pH 7,5, Mg SO4 5mM, gélatine 0,01 %) puis purifiés par un nouveau criblage réalisé dans les mêmes conditions.

12 clones ont ainsi été isolés et purifiés. L'ADN de chaque clone a été produit et purifié, selon la technique bien connue de l'homme de l'art, puis digéré par l'enzyme SalI qui permet d'isoler après électrophorèse sur gel d'agarose l'insert d'ADN génomique.

Le transfert sur membrane de nylon suivi d'une hybridation avec la sonde constituée de l'ADNc du clone 246, montre un signal positif, confirmant la présence dans cet ADN génomique d'une séquence complémentaire de l'ADNc 246.

Ces clones ont ensuite été cartographiés en utilisant une série d'enzymes de restriction. Un clone a été retenu parmi ceux-ci, qui présente un signal d'hybridation dans la région centrale du fragment inséré dans l'ADN phagique. Ce clone a été nommé 246C.

### Section 8 : Séquençage et analyse de la séquence du clone d'ADN génomique 246C

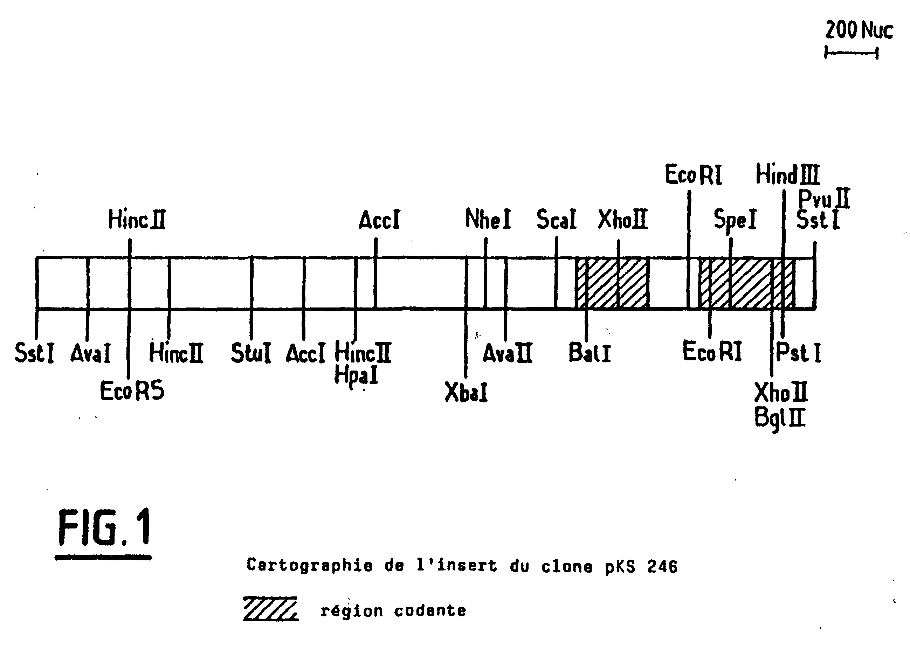

Un fragment de l'insert contenu dans ce phage a été isolé par digestion à l'aide de l'enzyme SstI, puis cloné dans le phage pBluescript ® 11 KS +/- (Stratagène) donnant le phagemide pKS246. Sa cartographie a été établie, elle est présentée sur la Figure 1. La séquence de cet insert [SEQ ID NO 7] a ensuite été déterminée par la méthode de Sanger et al (PNAS-USA, 14, 5463. 1977) après création de délétions progressives à l'aide des enzymes Exo III, mung bean et nucléase S1 selon la technique de Henikoff (Gene,-28, 351, 1984).

Cet insert, appelé gènes 246C, de 3046 paires de bases, est constitué d'une région codante de 853 pb débutant par un ATG en position 2146 et terminée par un codon TGA en position 2998 ; cette région est entrecoupée d'un intron commençant en position 2464 et terminant en position 2653. Trois sites potentiels d'initiation de la transcription ont été déterminés par extension d'amorce selon la technique décrite dans Sambrook et al. (opus cité, 1989), le site le plus probable est en position 2068.

La région promotrice du gène 246C en amont de la position 2146 est appelée promoteur 246C. L'étude de la séquence du promoteur 246C montre la présence de plusieurs motifs connus pour être impliqués dans la régulation des gènes.

Deux séquences consensus CAAT correspondant à cet élément régulateur de la transcription des gènes eucaryotes ainsi qu'une séquence complémentaire et inverse ATTG sont présentes aux positions 2051, 2101 et 1967.

Deux séquences consensus TATAA sont présentes aux positions 2089 et 2111 ainsi qu'une séquence complémentaire AATAT à la position 2020. Ces trois motifs sont tous situés entre 10 et 50 paires de bases en aval des actifs CAAT et 30 à 50 paires de bases en amont des sites de transcription potentiels.

Une séquence TGACG a été identifiée à la position 1950 ; cette séquence mise en évidence dans le promoteur 35S du CaMV, semble responsable de l'expression de gènes chimériques dans les racines et dans les feuilles (LAM et al. 1989, Proc Natl Acad Sci USA, 86, 7890).

Trois régions présentent une homogénéité importante avec les motifs HSE (Heat Shock Elements) des plantes (GURLEY et KEY, 1991, Biochemistry, 30. 1). Ces régions sont homologues avec la séquence consensus GAANNGAANNTTCNNTTC ou TTCNNTTCNNGAANNGAA ; elles sont proches des boites TATA et CAAT et dupliquées [SEQ ID NO : 17 et NO : 18].

Une séquence homologue au motif CCGTCC caractérisé comme étant impliqué dans la réponse à des éliciteurs d'origine fongique (LOIS et al, 1989, EMBO J., 8, 1641) a été localisée à la position 1822.

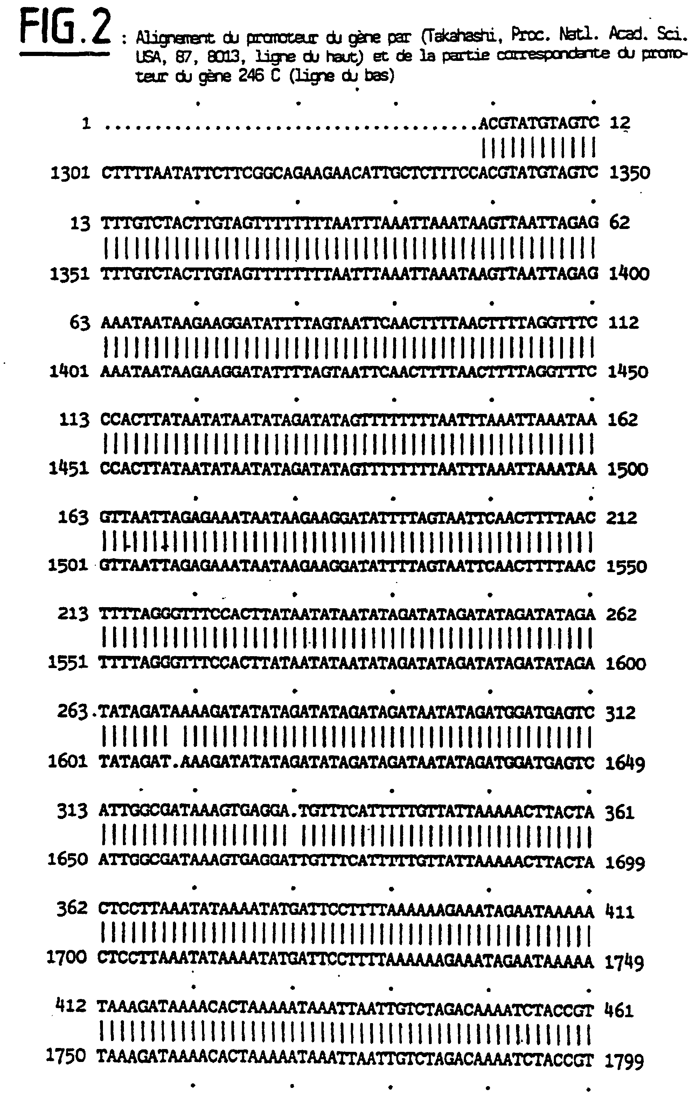

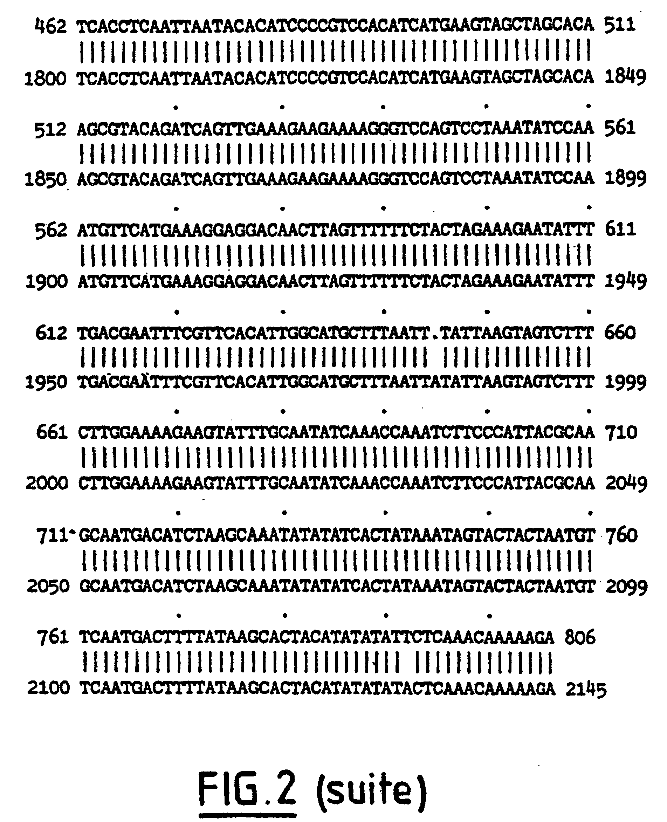

Le promoteur du gène 246 C présente sur environ 30% de sa longueur une homologie élevée (> à 90 %) sur 700 paires de bases avec le promoteur végétal isolé du tabac ayant la séquence [SEQ ID NO : 8] décrit par TAKAHASHI et al. 1990, Proc. Natl. Acad. Sci. USA vol. 87 pp 8013-8016. Sur la figure 2, on a représenté l'alignement de la séquence d'ADN de ce promoteur [SEQ ID NO : 9, 10 et 11] (ligne du haut) avec la partie correspondante de la séquence d'ADN du promoteur de l'invention [SEQ ID NO : 12 et 13] (ligne du bas).

### Section 9 : Construction du vecteur d'expression pSG123 associant le promoteur du gène 246C au gène de la β-glucuronidase.

A partir du phagemide pKS 246, la digestion par les enzymes HindIII et Ball permet d'isoler un insert de 2200 paires de bases environ, contenant la partie promotrice du gène 246C, le codon d'initiation de traduction et 11 codons codant pour la partie amino-terminale de la protéine.

Le plasmide pBI 101.3 (Clontech) est digéré par les enzymes HindIII et EcoRI ; le fragment de 2100 paires de bases environ, contenant le gène de la β-glucuronidase codée par le locus uidA de *E. coli*, suivi du terminateur de la nopaline synthase *d'Agrobacterium tumefaciens,* est ligué dans le plasmide pUC 19, ouvert aux mêmes sites, donnant un plasmide appelé plasmide pBI 201.3.

L'insert HindIII-BalI portant les séquences promotrices du gène 246C est cloné dans le plasmide pBI201.3 ouvert aux sites HindIII et SmaI.

Le vecteur obtenu appelé pSG 123 contient donc le gène de la glucuronidase sous contrôle du promoteur du gène 246C. La séquence nucléotidique du gène chimérique complet est la séquence [SEQ ID NO : 14].

### Section 10 : Protocole de l'expression transitoire dans des protoplastes de tabac du gène de la glucuronidase sous contrôle du promoteur du gène 246C.

### Préparation de protoplastes de tabac

Des feuilles de plantes de tabac (*Nicotiana tabacum*, var Samsun NN), âgées de 4 à 5 semaines sont prélevées, découpées en lanières et incubées dans du milieu T.O (tableau 1, adapté de Chupeau et al., 1974, C.R. Acad Sci (Paris), 278 D, 1565) contenant 1 g/litre de cellulase R 100 Onozuka, 200 mg/l de macerozyme Onozuka (Yakult Honsha, Nishinoniya, Japon) et 500 mg/l de pectolyase Y23 (Sheishin Pharmaceutical Ind. Japon) pendant 15 h à 22°C.

Les protoplastes sont séparés des débris cellulaires par tamisage sur un tamis de nylon de maille 85 µm suivi par une centrifugation de 5 min à 50 g sur une solution de saccharose à 19 % (poids/volume). Les protoplastes flottant sur ce milieu sont lavés une fois dans le milieu T.O, comptés et leur nombre ajusté à la densité de 1.5 x 10⁶ protoplastes/ml.

### Préparation du vecteur pSG 123

La souche *E. coli* contenant le vecteur pSG 123 est cultivée sur milieu Luria (Gibco) contenant 50 mg/l d'ampicilline. L'amplification du plasmide est réalisée selon le protocole décrit dans Sambrook et al., 1989 (opus cité). Le plasmide pSG 123 est ensuite purifié par centrifugation sur un gradient de chlorure de césium et par deux précipitations successives par l'éthanol. Le culot plasmidique est ensuite remis en solution dans du Tampon Tris-HCl 10 mM pH 8,0.

### Transformation par le polyéthylèneglycol

Les suspensions de protoplastes (320 µl par aliquote) sont incubées à 45°C pendant 5 min, puis rapidement refroidis sur la glace. Puis 50 µg de plasmide pSG123, 160 µl d'une solution de polyéthylèneglycol (40 % polyéthylèneglycol, 0,4 M mannitol, 30 mM MgCl₂, 0.1 % Mes pH 5.8) sont alors ajoutés. Au bout de 10 min, les protoplastes sont collectés par centrifugation et remis en suspension par agitation douce dans 500 µl de tampon T0 et incubés à l'obscurité & 28°C.

### Mesure de l'expression transitoire

Au bout de 24 h d'incubation. les protoplastes sont lysés après addition de 50 µl de tampon d'extraction 10X de la β-glucuronidase par une congélation à - 80°C suivie d'une décongélation à 37°C (Jefferson, 1987, Plant Molec. Biol. Reporter, 5, 387).

Le surnageant obtenu après centrifugation à 10 000 g est utilisé pour mesurer l'activité β-glucuronldase par fluorimétrie (Jefferson, 1987, Plant Molec. Biol. Reporter, 5, 387).

Parallèlement, la quantité de protéines présente dans les extraits est mesurée selon la méthode de Bradford à l'aide du kit Bio Rad(Bio Rad. Lab.).

**Tableau 1 :**

| Composition du milieu TO (pour 1 litre) | |
|---|---|
| NH₄ NO₃ | 825 mg |
| K NO₃ | 950 mg |
| CaCl₂, 2H₂O | 220 mg |
| Mg SO₄, 7H₂O | 185 mg |
| KH₂ PO₄ | 85 mg |
| H₃ BO₃ | 1 mg |
| Mn SO₄, 4H₂O | 100 µg |
| Zn SO₄, 7H₂O | 1 mg |
| Kl | 100 µg |
| AlCl₃ | 30 µg |
| NiCl₂, 6H₂O | 30 µg |
| CuSO₄, 5H₂O | 30 µg |
| Fe SO₄, 7H₂O | 27,8 mg |
| Na₂ EDTA, 2H₂O | 37,2 mg |
| Thiamine | 100 µg |
| Ac. nicotinique | 200 µg |
| Pyridoxine | 1 mg |
| Biotine | 10 µg |
| Panthoténate de Ca | 1 mg |
| Saccharose | 20 g |
| Inositol | 100 mg |
| Mannitol | 80 g |
| Acide 2-[N-Morpholino] ethanesulfonique (MES) | 200 mg |

pH ajusté à 5,8 avant autoclavage

### Section 11 : Expression transitoire du gène de la glucuronidase sous contrôle du promoteur du gène 246C dans des protoplastes de tabac infectés par Pseudomonas solanacearum.

Cette expression transitoire, déterminée selon le protocole de la section 10, est mesurée après incubation pendant 24 h des protoplastes préparés selon le protocole ci-dessus dans le milieu TO (décrit dans la section 10) contenant une suspension de bactéries *Pseudomonas solanacearum* (10 bactéries/protoplaste) obtenues comme décrit en section 1.

L'activité β-glucuronidase est exprimée en pmoles de méthylumbélliférone formée/min/mg de protéine.

Aucune activité n'est décelée dans les protoplastes de plantes n'ayant pas reçu d'ADN du vecteur pSG123. Une activité de 450 pmol/min/mg de protéine est mesurée sur des protoplastes traitées par du vecteur pBI221 (Clontech) contenant le gène de la β-glueuronidase sous contrôle du promoteur constitutif 35S CaMV ; cette activité est réduite à 300 pmol/min/mg de protéine après infection par les souches de *Pseudomonas solanacearum* GMI 1000 et GMI 1178.

Une activité de 600 pmol/min/mg de protéine est mesurée sur des protoplastes traités par du plasmide pSG 123 ; cette activité est peu modifiée après inoculation par la souche GMI 1178, elle augmente jusqu'à une valeur de 1000 pmol/min/mg de protéine après inoculation par la souche GMI 1000.

Le promoteur du gène 246C permet donc une expression basale importante du gène de la glucuronidase, expression plus importante que celle commandée par le promoteur 35S du CaMV, qui sert ici de contrôle. Ce promoteur présente de plus une forte inductibilité puisque l'expression de la β-glucuronidase augmente de 40 % après infection par la souche bactérienne GMI 1000.

### Section 12 : Expression transitoire du gène de la glucuronidase sous contrôle du promoteur du gène 246 C de protoplastes de tabac traités par un éliciteur (heptasaccharides de chitine) ou une hormone.

L'expression est mesurée après incubation des protoplastes pendant 24 h comme décrit ci-dessus dans le milieu TO contenant un éliciteur à la concentration de 25 µM ou une hormone, le 2-4-D (acide 2-4 dichlorophénoxyacétique) à la concentration 4 µM. Les éliciteurs utilisés (composés glycosidiques issus de la paroi des champignons pathogènes) sont des heptasaccharides de chitine ayant la propriété d'induire des réactions de défense dans les plantes (Roby et al., 1987, BBRC, 143, 885).

Aucune activité n'est détectée dans des protoplastes non traités et qui servent de contrôle. Une activité de l'ordre de 400 pmol de méthylumbélliférone formée/min/mg de protéine est mesurée à partir des protoplastes ayant reçu de l'ADN du vecteur pBI 221, cette activité n'est pas affectée par le traitement (éliciteurs ou hormone).

Les protoplastes ayant reçu de l'ADN du vecteur pSG 123 présentent une activité de 450 pmol/min/mg de protéine ; cette activité est accrue de 50 % si les protoplastes sont traités par l'hormone 2-4-D et de 70 % si les protoplastes sont traités par l'heptasaccharide de chitine.

Les caractéristiques de ce promoteur mises en évidence lors de l'infection de protoplastes par la souche bactérienne GMI 1000 (niveau de base élevé et inductibilité), peuvent être reproduites en utilisant un inducteur issu de la paroi de champignons phytopathogénes.

### Section 13 : Construction d'un vecteur d'expression stable dans les cellules végétales : le vecteur binaire pSG246

A partir du vecteur pSG 123, une coupure par les endonucléases de restriction HindIII et EcoRI, suivie d'une électrophorèse sur gel d'agarose, permet d'isoler le gène chimérique associant le promoteur 246C à la partie codante de la β-glucuronidase et le terminateur NOS. Le gène chimérique est introduit et ligué dans le vecteur binaire pBIN 19 (Clontech) préalablement ouvert aux sites HindIII et EcoRI donnant le vecteur pSG246. Ce vecteur binaire possède deux gènes de résistance à la kanamycine, l'un pouvant s'exprimer dans les bactéries, l'autre situé immédiatement en amont du gène recombinant complet (Bevan, 1984, Nucl. Ac. Res., 12, 8711) pouvant être transféré aux cellules végétales. Le gène de résistance à la kanamycine servira de marqueur de sélection au cours des étapes de transformation et d'analyse de la descendance des plantes transformées.

Le vecteur obtenu, appelé pSG246, est cloné dans la souche *E. coli* DH5 α.

### Section 14 : Transfert dans Agrobacterium du plasmide pSG246 contenant la β-glucuronidase sous contrôle du promoteur du gène 246C du tabac

### a) Transfert dans Agrobacterium tumefaciens

Ce transfert est réalisé par transformation selon la méthode de congélation-décongélation décrite dans Plant Molecular Biology Manual (Gelvin et al eds. , Kluwer Academic Publishers, 1988) et résumé ci-après.

Des cellules compétentes d'*Agrobacterium tumefaciens* (souche LBA 4404, Clontech) sont préparées, par refroidissement rapide dans la glace d'une culture en phase exponentielle de croissance. Les bactéries sont alors remises en suspension dans du CaCl₂ 20 mM. Des aliquotes de cette suspension sont distribuées dans des tubes Eppendorf, puis congelées dans l'azote liquide.

1 µg de plasmide pSG246 est ajouté aux cellules congelées, contenues dans un tube Eppendorf. La suspension est ensuite incubée à 37°C pendant 5 min ; 1 ml de milieu Luria (Gibco) est alors rajouté et le tube est incubé & 28°C pendant 4 h. Des parties aliquotes sont étalées sur des boites de Petri contenant un milieu minimum gélosé, décrit dans Plant Molecular Biology Manual (op. cité) en présence de 100 mg de rifan-picine et 25 mg/l de kanamycine. Dans ces conditions, seules poussent les colonies *d'Agrobacterium tumefaciens* ayant intégré le plasmide pSG246. Celles-ci contiennent le gène chimérique dans un contexte permettant sa replication.

La résistance aux deux antibiotiques des colonies sélectionnées est vérifiée en repiquant celles-ci sur le même milieu de sélection deux fois de suite. La présence du gène chimérique associent le promoteur 246 C à la partie codante de la β-glucuronidase dans *Agrobacterium tumefaciens* est vérifiée par la méthode de Southern Blot sur une préparation d'ADN total (Lyse des cellules, purification de l'ADN par extraction à l'aide du mélange phénol/chloroforme, selon le protocole décrit par Gelvin dans l'ouvrage cité ci-dessus, coupure de l'ADN purifié à l'aide d'enzymes de restriction, électrophorèse sur gel d'agarose, transfert sur membrane et hybridation selon les techniques bien connues de l'homme de l'art).

### b) Transfert dans Agrobacterium rhizogenes

Ce transfert est réalisé de la même façon que le transfert dans *Agrobacterium tumefaciens* décrit en a), avec la souche *Agrobacterium rhizogenes* A4 décrite par Guerche et al., (1987) Mol. Gen. Genet. 206, 382.

### Section 15 : Obtention de plantes de tabac transformées par Agrobacterium tumefaciens contenant le plasmide pSG246.

Le tabac *Nicotiana tabacum* cultivé in vitro a été infecté par *Agrobacterium tumefaciens* contenant le plasmide pSG246 selon la procédure de Horsch et al., bien connue de l'homme du métier (Horsch R.B. et al., 1985, Science 227, 1229-1231), dont les principales étapes sont exposées ci-après.

Des disques de feuilles de plantes axéniques de tabac *Nicotiana tabacum* (variété Bottom Spécial) sont incubés dans une culture d'A. *tumefaciens* hébergeant le plasmide pSG246. Les disques égouttés sur papier Whatman sont transférés sur des milieux de culture en boites de Pétri afin de multiplier les cellules transformées de façon à obtenir des cals (Murashige et Skoog, 1962, Physiol., Plant., 15, 473), puis produire des bourgeons en présence de céfotaxime (500 µg/ml) destinée à éliminer *Agrobacterium tumefaciens* et de kanamycine (100 µg/ml).

Parallèlement, des transformations ont été réalisées avec des souches d'*Agrobacterium tumefaciens* LBA 4404 contenant les vecteurs :
- pBI 101 (Clontech), constitué de la partie codante de la glucuronidase précédant le terminateur de la nopaline synthase dans le vecteur binaire pBIN 19. Cette construction, dénommée ci-après construction pBI 101, est dépourvue de promoteur.
- pBI 221 (Clontech), constitué d'un fragment de 800 paires de bases du promoteur 35S du virus de la mosaïque du choux fleur inséré devant la partie codante de la glucuronidase dans le vecteur pBI 101. Cette construction sera dénommée ci-après construction pBI 221.

Les bourgeons résistants à la kanamycine ont été ensuite transférés sur un milieu permettant l'induction des racines en présence de carbenicilline et de kanamycine. Les plantules sont ensuite repiquées en terrines dans un substrat composé de tourbe et de terreau et mises à croître en serre. Toutes les plantes transformées (génération RO) ayant survécu aux étapes de régénération et d'acclimatation en serre se sont révélées morphologiquement normales et fertiles. Elles ont été autofécondées et ont donné des graines (génération R1).

### Section 16 : Analyse de l'ADN génomique des plantes de tabac transformées par Agrobacterium tumefaciens contenant le plasmide pSG246 (génération RO), selon la technique de Southern Blot.

L'ADN génomique de tabac de haut poids moléculaire a été isolé à partir de feuilles matures de plantes transgéniques de la génération RO selon la méthode d'extraction à l'aide de bromure de cétyltriméthylammonium et de purification par précipitation, décrite dans l'ouvrage "Plant Molecular Biology Manual" déjà cité.

10 µg de cet ADN génomique ont été digérés pendant une nuit à 37°C avec 20 unités des enzymes de restriction HindIII et EcoRI. Les fragments de restriction obtenus ont été séparés par électrophorése sur gel d'agarose (1 %). L'ADN a été transféré selon la méthode de Southern Blot sur un filtre de Nylon (Hybond N⁺ Amersham), et hybridé avec une sonde nucléotidique comprenant une partie de la séquence du gène recombinant, marqué par couplage à la peroxydase (kit ECL, Amersham). Les membranes sont ensuite lavées et révélées selon le protocole recommandé par Amersham.
L'analyse des films permet de tirer les conclusions suivantes :
- certaines plantes ne possèdent pas de copies du gène recombinant transféré (absence de signal),
- la plupart des plantes testées contiennent au moins une copie sans réarrangement de la construction : promoteur 246C - séquence codante de la β-glucuronldsse-terminateur NOS, dénommée ci-après construction pSG 246
- certains profils suggèrent qu'il existe des réarrangements internes dans cette construction, mais ces événements sont rares.

### Section 17 : Etude des caractéristiques d'activation du promoteur 246 C dans les plantes de tabac transgéniques

Cette étude a été réalisée sur des plantes de la génération R1 qui ont été préalablement sélectionnées in vitro sur le milieu de Murashige et Skoog gélose renfermant 500 µg/ml de kanamycine.

10 plantes par descendance de transformant sélectionnées pour leur résistance à la kanamycine sont repiquées sur du terreau puis cultivées pendant 4 à 5 semaines en chambre de culture.

### a) Activation par la bactérie phytopathogène Pseudomonas solanacearum

### Inoculation par infiltration

Des tests d'inoculation sont conduits selon le protocole décrit en section 1 sur quatre feuilles appartenant à 2 plantes différentes. Les mesures d'activité glucuronidase effectuées selon la méthode décrite par Jefferson (Plant Molecular Biology Reporter, 5, 387, 1987) en utilisant le 4-méthylumbelliféryl β-D-glucuronide comme substrat.
Les résultats montrent que :
- les plantes renfermant la construction pBI 101 ne présentent pas d'activité glucuronidase détectable,
- les plantes renfermant la construction pBI 121 présentent une activité glucuronidase comprise entre 5000 et 70 000 pmoles de méthylumbélliférone/min/mg de protéine correspondant à une expression constitutive attendue pour cette construction. Une légère activation de ce promoteur en réponse au stress d'infiltration a été constatée pour certains transformants,
- les plantes contenant la construction pSG246 présentent une activité glucuronidase faible dans les plantes non inoculées, comprise entre 2000 et 5000 pmol/mg de protéine. Une forte induction est mesurée en réponse à l'infection bactérienne, et ce quelle que soit la souche bactérienne utilisée (GMI 1000 ou K60 (Sequeira et al, Physiol. Plant. Pathol., 10, 43, 1977). souche compatible provoquant des symptômes). Le facteur d'induction (rapport entre l'activité mesurée après inoculation et l'activité mesurée avant inoculation) est de l'ordre de 20 fois et les valeurs d'activité dépassent parfois celles obtenues avec les plantes exprimant la construction pBI 121.

### Infection bactérienne localisée :

Une infection bactérienne localisée est réalisée par dépôt d'une goutte de suspension bactérienne de *Pseudomonas solanacearum* (3 µl renfermant 3 x 10⁵ bactéries obtenues comme décrit en section 1) sur une blessure obtenue par perforation d'une feuille à l'aide d'une aiguille de seringue.

Le promoteur 246C est activé autour de la lésion créée par l'Infection et également dans toutes les parties de la plante infectée (feuille inoculée, feuilles supérieures et feuilles inférieures de la plante et racines). Cette activation systémique se produit dès les premières heures après Inoculation.

Aucune activation n'a été observée dans des plantes de génération R1 issues de plantes transformées par les constructions pBI 101 et pBI 121.

Le même type d'infection localisée, réalisée au niveau de racines de plantes de tabacs cultivées in vitro conduit également à une activation forte du promoteur au site d'inoculation mais également dans l'ensemble de la racine et dans la partie aérienne de la plante.

### b) Activation par le champignon pathogène Chalara elegans

### Etude in vitro

10 à 15 ml de milieu Murashige et Skoog liquide sont versés dans une boite de Petri contenant une culture de 3 semaines de *Chalara elegans* en cours de sporulation (culture sur milieu gélosé Potato dextrose Agar PDA, Difco).

Le grattage de la surface de la culture permet de recueillir les spores. Après comptage, une dilution appropriée permet d'obtenir des suspensions de 10⁴ et 10⁵ spores par ml.

Des aliquotes de 7 ml sont distribuées dans des boites Magenta (sigma). Une plante de tabac transgénique (âgée de 3 semaines environ et cultivée en milieu stérile) transformée par le gène de β-glucuronidase sous contrôle du promoteur 246C est introduite dans chaque boîte, leurs racines trempant dans la suspension de spores. Les plantes sont ensuite prélevées, congelées et l'activité glucuronidase déterminée. Au moment de l'infection l'activité spécifique est de- 20 000 pmoles de méthylumbélliférone par mg de protéine. Comparativement à des témoins non inoculés placés dans les mêmes conditions, cette activité est multipliée par des facteurs de 8 et 8,5 pour les infections à 10⁴ et 10⁵ spores par ml, respectivement, dès 4 jours après l'inoculation.

### Etude en serre

10 plantes par descendance de transformant, sélectionnées pour leur résistance à la kanamycine sont repiquées en godets de dimension 3 x 3 cm. A l'apparition de la 5éme feuille, les plantes sont inoculées en déposant au niveau du collet une suspension de 5 x 10⁵ endoconidies de *Chalara elegans.*

### c) Activation par le champignon pathogène Sclerotinia sclerotiorum

### Etude sur disques foliaires

Des disques foliaires de 20 mm de diamètre provenant de plantes exprimant la construction pSG246 sont placées en survie sur un milieu liquide approprié. Sur chacun de ces disques est placé un cube de gélose de 5mm de côté environ et contenant du mycélium de *Sclerotinia Sclerotiorum*. L'activité glucuronidase, mesurée en fonction du temps révèle que la présence de mycélium induit l'activité glucuronidase après 7 heures de contact. Au bout de 24 heures, l'activité est multipliée par 4 par rapport à celle de disques foliaires non mis en contact avec le mycélium. La même expérience réalisée sur des disques de feuille issus de tabacs témoins (n'exprimant pas la construction pSG246) ne révèle qu'une activité glucuronidase assimilable à un bruit de fond.

### d) Activation par des éliciteurs

Deux types d'éliciteurs ont été utilisés l'un de type biotique correspondant à des molécules issus de molécules ou de macromolécules naturelles, l'autre de type abiotique correspondant à des molécules chimiques.

### d1. Eliciteurs biotiques d'origine bactérienne ou fongique

Les élicileurs utilisés sont l'harpin qui est une protéine isolée de *Erwinia amylovora* et une protéine isolée du surnageant de culture de *Pseudomonas solanacearum.* De même des éliciteurs protéiques tels que la cryptogéine isolée de *Pseudomonas cryptogea* et la capsicéine isolée de *Pseudomanas capsict* (Ricci et al. 1989, Eur. J. Biochem. 183 ; 555-563) ont été testés.

Dans tous les cas, des concentrations appropriées d'éliciteurs ont été infiltrés à l'aide d'une seringue sous l'épidémie inférieur des feuilles de tabac exprimant la construction pSG246. L'induction d'activité, dans tous les cas est induit par la présence d'éliciteur dans un anneau de 5mm environ immédiatement adjacent à la zone d'infiltration. La stimulation est très apparente 24 heures après l'infiltration mais visible dès 6 heures. L'activité au bout de 24 heures est souvent multipliée par 5 à 6 par comparaison à celles des plantes témoin (plantes exprimant la construction pSG246 mais sans éliciteurs injectés sous l'épiderme).

### d2. Eliciteurs abiotiques : acide salicylique, sulfate de cuivre

Des feuilles de tabac exprimant la construction pSG246 et détachées de la plante sont immergées dans des solutions de concentration 1 à 10 µg/ml d'acide salicylique ou de 0,025 à 0.25 mM de sulfate de cuivre.

En présence d'acide salicylique l'activité glucuronidase est multipliée par 5 au bout de 6 heures et par 10 après 24 heures. Dans le cas du sulfate de cuivre, l'activité glucuronidase est multipliée par 17 à 0,025 mM et 11 à 0,25 mM si l'on compare à des feuilles de plantes témoins non traitées.

### d3. Induction par des régulateurs de croissance

L'application d'une auxine sous la forme d'acide 2,4-dichlorophénoxyacétique (2,4-D) a été réalisée aux concentrations de 1,5 et 10 µM. L'application réalisée par immersion de pétioles des feuilles détachées de tabac exprimant la construction pSG246 montre que l'induction dans la feuille commence 6 heures après l'application de l'auxine à la concentration de 5 µM, pour être stimulée 18 fois après 12 heures comparée aux feuilles de plantes témoins, non traitées par l'auxine.

L'activité glucuronidase est mesurée lors de l'apparition des symptômes de la maladie soit 15 jours environ après l'inoculation.

Les résultats de l'activité mesurée au niveau de la partie aérienne de la plante entière montrent que :
- les plantes renfermant la construction pBI 101 ne présentent pas d'activité glucuronidase détectable,
- les plantes transformées par la construction pBI 121 présentent une activité de 12 000 à 60 000 pmoles de méthylumbélliférone/min/mg de protéine. Cette activité varie peu après inoculation,
- les plantes transformées à l'aide de la construction pSG246 présentent une activité glucuronidase faible chez les plantes saines. Cette activité augmente considérablement dans les plantes présentant des symptômes, atteignant des vecteurs de 85 000 pmoles de méthylumbelliferone formée/min/mg de protéine.

### e) Activation par une blessure

Cette activation a été recherchée sur des feuilles de plantes de génération R1 contenant la construction pSG246, âgées de 5 semaines environ et préalablement sélectionnées pour leur résistance à la kanamycine.

L'excision simple d'une feuille entraîne une activation lente et faible du promoteur, à la fois dans la feuille et dans la plante ; cependant, la lacération d'une feuille entraîne une augmentation très forte (5 fois) et extrêmement rapide (30 min) de l'activité glucuronidase de la feuille lacérée.

### f) Activation par un choc thermique

Des plantes de tabac, de génération R1, contenant la construction pSG246, âgées de 5 semaines environ et préalablement sélectionnées pour leur résistance à la kanamycine sont transférées pendant 2 ou 4 h dans une enceinte à 40°C afin de provoquer un choc thermique. A la fin du traitement, les plantes sont immédiatement congelées dans l'azote liquide et leur activité β-glucuronidase déterminée sur l'ensemble de la partie aérienne.

Le même protocole est appliqué à des plantes transformées par la construction pBI 121 ; l'activité de ces dernières n'est pas modifiée par le choc thermique.

Par contre, les plantes contenant la construction pSG246 présentent une forte augmentation d'activité glucuronidase après le choc thermique ; le facteur moyen de stimulation, déterminé sur plusieurs plantes, est voisin de 12.

### g) Expression au cours du développement et répartition spatiale de l'expression dans la plante

L'utilisation du substrat histochimique de révélation de l'activité glucuronidase, le 5-brome-4-chloro-3-indolyl-β-D-glucuronide de cyclohexylammonium (X-gluc) selon la méthode décrite par Jefferson (Plant Molecular Biology Reporter, 5, 387, 1987), permet de visualiser la localisation de l'activité dans les tissus de la plante.

### Germination des graines :

Au cours de la germination des graines de tabac de génération R1 exprimant la glucuronidase sous contrôle du promoteur 246C, l'expression est non détectable dans les cotylédons, élevée dans toute la racine et très forte dans les méristèmes racinaires et caulinaires. Dans la plante développée la détection de l'activité glucuronidase a été relevée dans tous les tissus testés, y compris dans la graine sèche.

### Section 18 : Obtention de plantes de colza transformées par Agrobacterium rhizogenes contenant le plasmide pSG246.

La transformation est réalisée selon le protocole de P. Guerche et al. (P. Guerche et al., 1987, Mol. Gen. Genet., 206, 382). Les différents milieux de culture sont ceux décrits par Pelletier et al. (Pelletier et al., 1983, Mol. Gen. Genet., 191, 244). Leur composition sera explicitée par la suite (tableau 2).

### a) Obtention de racines transformées

Des segments de tige sont prélevés sur l'extrémité apicale de plantes de colza (*Brassica napus* : variétés de printemps Brutor et Westar et variété d'hiver) de 1 m de haut environ. Ces segments sont stérilisés en surface, rincés dans de l'eau stérile, découpés en segments de 1,5 cm de long environ et placés dans un tube contenant le milieu A.

L'inoculation de l'extrémité de ce segment est effectuée par dépôt d'une suspension de la souche d'*Agrobacterium rhizogenes* contenant le plasmide psG 246.

Des racines transformées apparaissent sur le segment de tige au bout de 1 à 2 semaines, elles sont prélevées et placées sur le milieu B gélosé (15 g/l) et complémenté par 500 µg de céfotaxime/ml.

### b) Régénération de plantes transformées

Des fragments de racines sont incubés pendant 15 jours dans le milieu D contenant 3 mg/l d'acide 2,4-dichlorophénoxyacétique, puis placés sur le milieu RCC d'induction de bourgeons. Des plantes racinées sont ensuite obtenues par passage des bourgeons sur les milieux F et G.

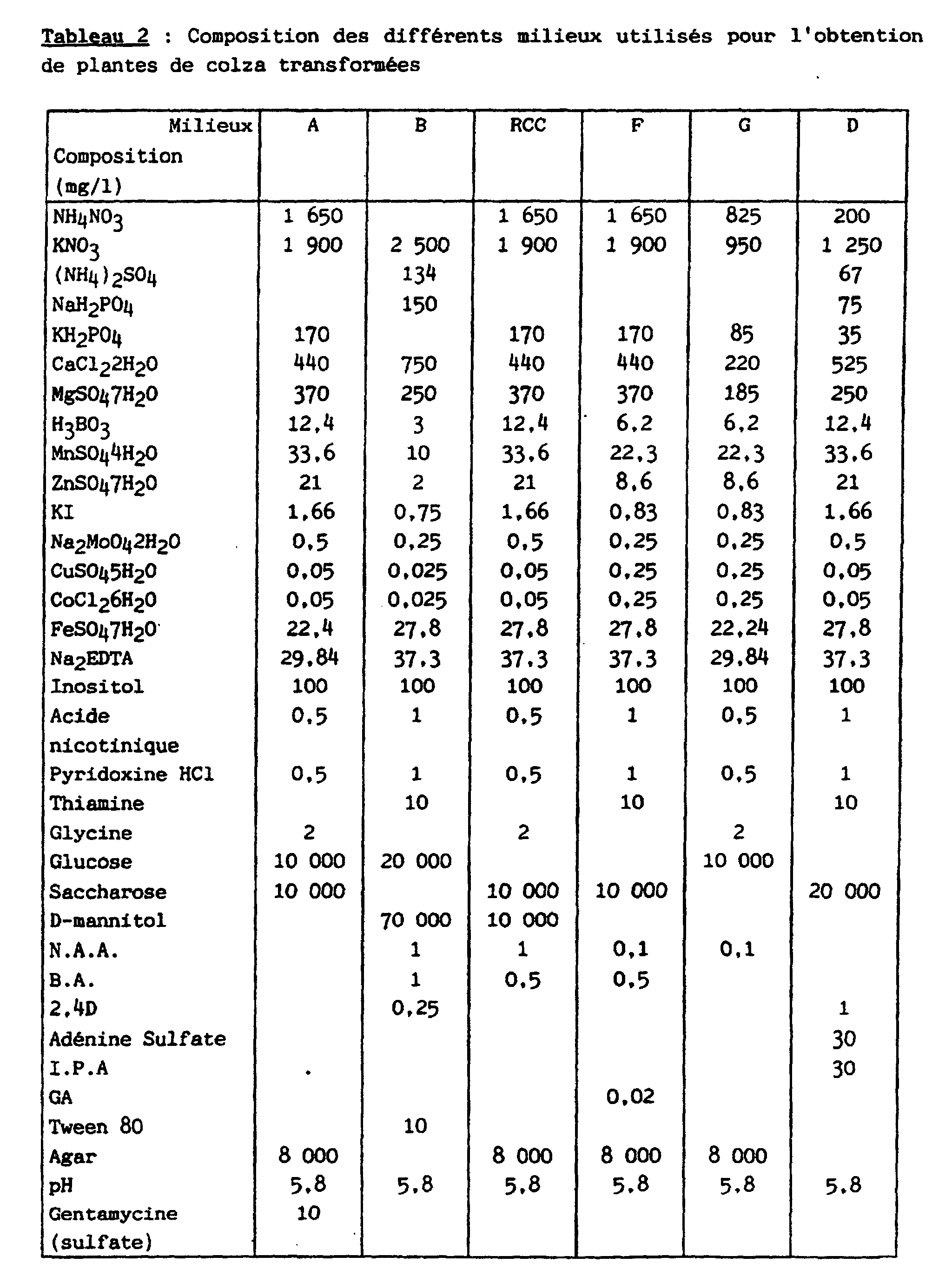

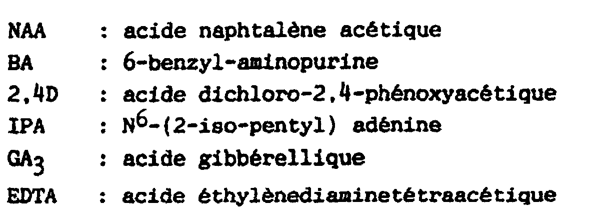

### Section 19 : Caractéristiques de l'expression du gène de glucuronidase sous contrôle du promoteur 246C dans les plantes de colza

### a) Activation par une blessure

Les feuilles de plantes de colza de génération R1, renfermant la glucuronidase sous contrôle du promoteur 246C ont été excisées ou excisées et lacérées.

Une activation faible d'activité glucuronidase est observée dans les feuilles excisées ; une activation très forte (6 fois l'activité basale) et extrêmement rapide (30 min environ) est consécutive à la lacération.

### b) Infection par les champignons phytopathogènes Parasite foliaire (Alternaria brassicae) :

Des plantes de colza de génération R1, possédant la construction pSG246 sont cultivées pendant 3 semaines environ en pot sur du terreau. Les plantes sont alors inoculées localeaent à l'aide d'une suspension de spores (10 ml, contenant 1000 spores obtenues après culture sur le milieu gélosé PDA) du champignon pathogène *Alternaria brassicae*, déposées sur une blessure réalisée à l'aide d'une aiguille. Une nécrose se développe alors autour de cette blessure.

On observe alors une forte stimulation de l'activité glucuronidase (détectée par test à l'aide de X-gluc, Section 17e) dans la feuille inoculée, autour de la zone nécrotique.

### Parasite racinaire (Rhizoctonia solani)

Des graines de colza de génération R1, transformées par la construction pSG246 sont semées sur un substrat constitué d'un mélange de tourbe, vermiculite et sable (10: 10: 5. v : v) contenu dans un pot de 1 litre. Les graines sont recouvertes d'une couche de 1 cm de substrat contenant 10 000 propagules viables de *Rhizoctonia solani* par gramme. Ces propagules sont obtenus par culture pendant 15 jours d'une souche de *Rhizoctonia* en fiole de Roux sur des grains de riz, suivi d'un broyage à une granulométrie inférieure à 1 mm.

Au cours de leur développement, les plantes de colza sont attaquées au niveau racinaire.

On observe une forte stimulation de l'activité glucuronidase (détectée par test à l'aide de X-gluc, Section 17e) dans les racines de plantes infectées, comparativement à l'activité mesurée dans les racines de plantes contrôles (même descendance R1 de plantes transformées mais non infectées). De plus, une forte stimulation de l'activité glucuronidase est induite de façon systémique dans les parties aériennes.

### c) Activation par un choc thermique des plantes de colza de génération R1

Des plantes de colza, de génération R1, contenant la construction pSG246, âgées de 5 semaines environ sont transférées pendant 2 ou 4 h dans une enceinte à 40°C afin de provoquer un choc thermique. A la fin du traitement, les plantes sont immédiatement congelées dans l'azote liquide et leur activité β-glucuronidase déterminée sur l'ensemble de la partie aérienne.

Le même protocole est appliqué à des plantes transformées par la construction pBI 121 ; l'activité de ces dernières plantes n'est pas affectée par le choc thermique.

Par contre, les plantes contenant la construction pSG246 présentent une forte augmentation d'activité glucuronidase après le choc thermique ; le facteur moyen de stimulation, déterminé sur plusieurs plantes, est voisin de 12.

### d) Expression au cours du développement

Au cours de la germination de graines de colza de génération R1 renfermant la glucuronidase sous contrôle du promoteur 246C. on observe une expression de cette protéine dans les cotylédons ; une expression élevée dans toute la racine et très forte dans les méristèmes racinaires et caulinaires.

Une très forte expression a également été mesurée dans les racines et cals transformés, au cours des étapes de régénération de plantes transgéniques ainsi que dans les diverses parties de la plante (y compris les graines matures).

### Section 20 : Obtention de cals de tournesol transformés par Agrobacterium rhisogenes contenant le plasmide pSG246.

La transformation est réalisée selon le protocole de Guerche et al (Mol. Gen. Genet., 206, 382, 1987) initialement mis au point pour la transformation du colza. Les différents milieux de culture sont ceux décrits par Pelletier et al. (Mol. Gen. Genet. 191, 244, 1983) leur composition a été explicitée (tableau 2).

Des hypocotyles de tournesol sont obtenus par germination pendant 7 à 10 jours de graines sur de la vermiculite. Ces graines sont placées dans une chambre de culture, les conditions 16 h d'éclairement à 20°C/8 h d'obscurité à 17°C. Les hypocotyles sont stérilisés en surface, rincés dans de l'eau stérile et placés dans un tube contenant du milieu Murashige et Skoog, dont la concentration en macroéléments est réduite de moitié.

L'inoculation de l'extrémité de ce segment est effectuée par dépôt d'une suspension de la souche *d'Agrobacterium rhisogenes* contenant le plasmide pSG246.

Des racines transformées apparaissent au bout de un mois, elles sont prélevées et placées sur le milieu B gélose (15 g/l) et complémenté par 500 µg de cefotaxime/ml pendant 4 semaines avec un repiquage hebdomadaire. Elles sont ensuite cultivées dans le même milieu liquide en agitation (100 tours/min) et repiquées tous les mois. Le passage de ces racines dans le milieu D, permet la formation de cals à partir des racines transformées.

L'activité glucuronidase des racines cultivées dans le milieu B liquide et de cals cultivés sur le milieu D, estimée par fluorimétrie, présente des valeurs très importantes, comprises entre 10⁴ et 10⁵ pmoles de methylumbelliferone formée/min/mg de protéines.

### Section 21 : Expression transitoire du gène de la glucuronidase sous contrôle du promoteur du gène 246C dans des embryons immatures de Tournesol.

Les embryons immatures des plantes mères au champ du génotype 105 ont été prélevés et mis en culture pendant 14 jours sur le milieu I (tableau 3) à 25° C et à l'obscurité. Ces embryons sont ensuite cultivés pendant 3 jours sur le mileu II à 25° C sous une photopériode de 16 heures par jour/ 8 heures par nuit. Une vingtaine d'embryons sont ensuite déposés côte-à-côte sur le milieu III.

### Préparation du vecteur pSG123 :

Celle-ci est réalisée selon le protocole décrit en Section 10.

### Transformation du matériel végétal :

L'introduction de l'ADN plasmidique (vecteur pSG123) dans les cellules végétales est réalisé par utilisation du canon à particules construit sur le principe décrit par Zumbrunn (Zumbrunn et al. 1989 Technique 1(3) 204-216). L'ADN plasmidique est adsorbé sur des nicroparticules de tungstène à raison de 4µg/mg de tungstène. 2,5mg du mélange tungstène/ADN sont alors déposés sur un macroprojectile qui est accéléré par l'explosion d'une cartouche.

L'écrasement du macroprojectile sur une plaque d'arrêt percée d'un trou permet de projeter les microparticules de tungstène et l'ADN dans les cellules.

### Mesure de l'expression transitoire :

L'ADN adsorbé sur les microparticules de tungstène qui ont pénétré dans les cellules végétales est libéré ; le gène chimérique associant le promoteur du gène 246C à la glucuronidase est alors transcrit puis traduit. La glucuronidase obtenue est alors visualisée par le test histochimique décrit par JEFFERSON et al, 1987 Plant Molecular BIology Reporter 5,387, en utilisant le substrat 5-bromo-4-chloro-3-indolyl-β-D-glucuronide de cyclohexylammonium (X-gluc). Les cellules exprimant le gène présentent alors une coloration bleue.

Le comptage du nombre de cellules bleues obtenues par boite de Petri au cours d'une expérience d'expression transitoire permet de compter le nombre de cellules transformées et d'estimer l'expression de la construction chimérique testée.

L'expression transitoire mesurée en utilisant le substrat X-gluc., 48 heures après le bombardement à l'aide du canon à microparticules montre que le gène de β-glucuronidase s'exprime dans les embryons immatures de Tournesol.

L'intensité est plus forte que celle induite par l'utilisation du plasmide pBI221 (Clontech), dans lequel le gène de glucuronidase est placé sous contrôle du promoteur 35S du virus de la mosaïque du choux-fleur.

Le nombre de cellules transformées exprimant le gène de glucuronidase sous contrôle du promoteur du gène 246C est voisin de 140 par boite (valeur moyenne de 4 expériences) alors que le nombre de cellules transformées est de 60 par boite (valeur moyenne de 4 expériences) en présence du plasmide pBI221 dans lequel le gène de glucuronidase est placé sous contrôle du promoteur 35 S du virus de la mosaïque du choux-fleur.

Les embryons sont ensuite égouttés sur du papier filtre stérile, puis remis en culture sur le milieu II à l'obscurité pendant 3 jours. Les embryons sont alors brièvement rincés par du milieu Murashige et Skoog liquide (Murashige et Skoog, 1962, Physiol. Plant 15 : 473) contenant 500 mg/l de l'antibiotique céfotaxime. Ils sont ensuite égouttés sur du papier filtre stérile et mis en culture sur du milieu III contenant 250 mg/l de céfotaxime. 250 mg/l de carbenicilline et 50 mg/l de paromomycine. Cette culture s'effectue à 25°C sous une photopériode de 16 h jour / 8 h nuit ; les tissus végétaux sont repiqués tous les 21 jours sur ce même milieu.

Les bourgeons néoformés à partir de ces tissus sont transférés sur le milieu IV, sous les mêmes conditions de température et photopériode. Les plantes enracinées sont ensuite mises à pousser en serre.

### Section 22 : Expression de la β-glucuronidase, sous contrôle du promoteur 246C dans les plantes de Tournesol transformées.

Les plantes exprimant la construction pSG246 ont une expression de la glucuronidase au moins égale à celle obtenue avec des plantes transformées à l'aide de la construction pBI121.

**Tableau 3 :**

| Composition des différents milieux utilisés pour l'obtention de plantes de tournesol transformées | | | | |
|---|---|---|---|---|
| Milieux | I | II | III | IV |
| KNO₃ | 2500 | 2500 | 1900 | 1900 |
| NH₄ NO₃ | - | - | 1650 | 1650 |
| CaCl₂, 2H₂O | 150 | 150 | 440 | 440 |
| MgSO₄,7H₂O | 250 | 250 | 370 | 370 |
| KH₂ PO₄ | - | - | 170 | 170 |
| (NH₄)₂ SO₄ | 134 | 134 | - | - |
| NaH₂PO₄,H₂O | 150 | 150 | - | - |
| ZnSO₄,7H₂O | 2 | 2 | 8,6 | 8,6 |
| H₃ BO₃ | 3 | 3 | 6,2 | 6,2 |
| KI | 0,75 | 0,75 | 0,83 | 0,83 |
| CuSO₄,5H₂O | 0,025 | 0,025 | 0,025 | 0,025 |
| Na₂MoO₄,2H₂O | 0,25 | 0,25 | 0,25 | 0,25 |
| CoCl₂, 6H₂O | 0,025 | 0,025 | 0,025 | 0,025 |
| Mn SO₄, 4H₂O | 10 | 10 | 22,3 | 22,3 |
| Na₂ EDTA | 37,3 | 37,3 | 37,3 | 37,3 |
| FeSO₄ ,7H₂O | 27,8 | 27,8 | 27,8 | 27,8 |
| Ac. nicotinique | 1 | - 1 | 0,5 | 0,5 |
| Thiamine HCl | 10 | 10 | 0,1 | 0,1 |
| Pyridoxine HCl | 1 | 1 | 0,1 | 0,5 |
| Myo-inositol | 4000 | 4000 | 100 | 100 |
| L-glycine | - | - | - | 2 |
| L-alanine | 1000 | 1000 | - | - |
| L-glutamine | 800 | 800 | - | - |
| L-serine | 160 | 160 | - | - |
| L-tryptophane | 50 | 50 | - | - |
| L-cystéine | 10 | 10 | - | - |
| Ca-D-panthoténate | - | - | 0.8 | - |
| Ac. folique | - | - | 0.1 | - |
| Chl. de choline | - | - | 0,1 | - |
| Ac p-Aminobenzoïque | - | - | 0.05 | - |
| Riboflavine | - | - | 0.05 | - |
| Saccharose | 120 000 | 60 000 | 30 000 | 30 000 |
| Ac.Dichloro-2-4-phenoxyacétique | 2 | - | - | - |
| 6-Benzylaminopurine | - | 0.4 | - | - |
| Kinétine | - | - | 1 | - |
| Ac.indoleacetique | - | - | - | 0,05 |
| Agar | 7000 | 7000 | 7000 | 8000 |
| pH | 5.7 | 5.8 | 5.7 | 5.7 |

### Section 23 : Protocole de l'expression dans les tissus de monocotylédones du gène de la β-glucuronidase, sous contrôle du promoteur 246C

### Obtention du matériel végétal :

Des graines du génotype d'orge GERBEL sont mises à germer en serre sur de la vermiculite. Au bout de 7 jours, les feuilles et les racines ont été prélevées et placées sur une boite de Petri contenant du milieu Murashige et Skoog gélosé pour les expériences d'expression transitoire.

Des embryons immatures de Maïs sont prélevés 10 à 14 jours après pollinisation à partir de pieds mères (lignée LH132) cultivés en serre. Les embryons sont placés côté axe embryonnaire en contact avec le milieu d'induction (composition donnée dans le tableau 4 ci-après) puis sur le milieu d'entretien (tableau 5), 3 semaines plus tard. Les cals obtenus sont repiqués toutes les semaines. Les expériences d'expression transitoire sont réalisées quelques heures après le repiquage.

### Préparation du vecteur pSG123 :

Celle-ci est réalisée selon le protocole décrit en Section 10.

### Transformation du matériel végétal :

L'introduction de l'ADN plasmidique (vecteur pSG123) dans les cellules végétales est réalisé par utilisation du canon à particules construit sur le principe décrit par Zumbrunn (Zumbrunn et al. 1989 Technique 1 (3) 204-216). L'ADN plasmidique est adsorbé sur des microparticules de tungstène à raison de 4 µg/mg de tungstène. 2,5 mg du mélange tungstène/ADN sont alors déposés sur un macroprojectile qui est accéléré par l'explosion d'une cartouche.

L'écrasement du macroprojectile sur une plaque d'arrêt percée d'un trou permet de projeter les microparticules de tungstène et l'ADN dans les cellules.

### Mesure de l'expression transitoire :

L'ADN adsorbé sur les microparticules de tungstène qui ont pénétré dans les cellules végétales est libéré ; le gène chimérique associant le promoteur du gène 246C à la glucuronidase est alors transcrit puis traduit. La glucuronidase obtenue est alors visualisée par le test histochimique décrit par JEFFERSON et al, 1987 Plant Molecular Biology Reporter 5,387, en utilisant le substrat 5-brome-4-chloro-3-indolyl-β-D-glucuronide de cyclohexylammonium (X-gluc). Les cellules exprimant le gène présentent alors une coloration bleue.

Le comptage du nombre de cellules bleues obtenues par boîte de Petri au cours d'une expérience d'expression transitoire permet de compter le nombre de cellules transformées et d'estimer l'expression de la construction chimérique testée.

**TABLEAU 4 :**

| Milieu d'induction de cals de Maïs à partir d'embryons immatures (en mg pour 1 litre) | |
|---|---|
| MgSO₄, 7H₂O | 370 |
| CaCl₂, 2H₂O | 440 |
| KNO₃ | 1 900 |
| NH₄NO₃ | 1 650 |
| KH₂PO₄ | 170 |
| Na₂MoO₄, 2H₂O | 0,25 |
| CuSO₄, 5H₂O | 0,025 |
| MnSO₄, H₂O | 16,75 |
| H₃BO₃ | 6,2 |
| ZnSO₄, 7H₂O | 8,6 |
| KI | 0,83 |
| CoCl₂, 6H₂O | 0,025 |
| FeEDTA | 65,1 |
| Saccharose | 20 000 |
| Hydrolysat de caséine | 100 |
| L-proline | 5 800 |
| Glycine | 2 |
| Acide nicotinique | 0,5 |
| Pyridoxine HCl | 0,5 |
| Inositol | 100 |
| Thiamine HCl | 0,1 |
| Acide abscissique | 0,06 |
| Chloramben | 4,12 |
| Phytagel | 3000 |
| pH = 5.7 Autoclavage 20 min à 120°C | |

**TABLEAU 5**

| Milieu d'entretien des cals de Maïs (en mg pour 1 litre) | |
|---|---|
| MgSO₄, 7H₂O | 370 |
| CaCl₂, 2H₂O | 440 |
| KNO₃ | 1 900 |
| NH₄NO₃ | 1 650 |
| KH₂PO₄ | 170 |
| Na₂MoO₄, 2H₂O | 0,25 |
| CuSO₄, 5H₂O | 0,025 |
| MnSO₄, H₂O | 16,75 |
| H₃BO₃ | 6,2 |
| ZnSO₄, 7H₂O | 8,6 |
| KI | 0,83 |
| CoCl₂, 6H₂O | 0,025 |
| FeEDTA | 65,1 |
| Saccharose | 20 000 |
| Hydrolysat de caséine | 100 |
| L-proline | 2 900 |
| Glycine | 2 |
| Acide nicotinique | 0,5 |
| Pyridoxine HCl | 0,5 |
| Inositol | 100 |
| Thiamine HCl | 0,1 |
| Dicamba (Banvel®) | 0,002 |
| Gelrite | 3 000 |
| pH = 5,7 Autoclavage 20 min à 120°C | |

### Section 24 : Expression transitoire du gène de la β-glucuronidase sous contrôle du promoteur du gène 246C dans les tissus d'orge et les cals de Maïs

L'expression transitoire mesurée en utilisant le substrat X-gluc, 48 heures après le bombardement à l'aide du canon à microparticules montre que le gène de β-glucuronidase s'exprime dans les feuilles et les racines d'orge et également dans les cals de Maïs.

L'intensité de l'expression est aussi forte que celle induite par l'utilisation du plasmide pBI 221 (Clontech), dans lequel le gène de glucuronidase est placé sous contrôle du promoteur 35S du virus de la mosaïque du choux-fleur.

Le promoteur du gène 246C du tabac est donc capable de diriger l'expression d'un gène dans les monocotylédones.

### Section 25 : Construction d'un plasmide plaçant le gène de la chitinase tomate-tabac sous le contrôle du promoteur inductible et expression de celui-ci dans le tabac

### a) Préparation de la séquence promotrice

Le plasmide pSG123 décrit précédemment est digéré à l'aide des endonucléases Hind III et Sca I. Après électrophorèse sur gel d'agarose, le fragment Hind III - Sca I de 2088 paires de bases contenant tout le promoteur inductible à l'exception des 57 paires de bases situées immédiatement en amont de l'ATG est purifié.

La ligation de ce fragment purifié, de l'oligonucléotide de synthèse Sca I - Bam HI de 62 paires de bases de séquence [SEQ ID NO : 15] et d'un vecteur pTZ 19R (Pharmacia) linéarisé grâce aux endonucléases Hind III et Bam HI a donné naissance au plasmide pPH 111.

A partir de ce plasmide pPH 111 par coupure à l'aide des endonucléases Hind III - Bam HI, puis électrophorèse sur gel d'agarose, le fragment Hind III - Bam HI de 2150 paires de bases contenant le promoteur inductible dans son entier est isolé.

### b) Préparation du fragment portant un gène hybride codant pour une protéine à activité endochitinase

Le fragment BamHI - EcoRI provenant du plasmide pBR1 décrit dans la demande de brevet EP-493 581, Exemple 1 et contenant un gène chimérique codant pour une protéine à activité endochitinase [SEQ ID NO : 16], qui comprend la séquence codant pour une endochitinase hybride tomate-tabac (en position 438-1587) et le terminateur NOS, est purifié.

### c) Clonage dans le vecteur binaire pBIN 19

On a ligué à l'aide de l'ADN ligase T4 la séquence promotrice (cf. ci-dessus) la séquence codant pour la chitinase et la séquence terminatrice. dans le vecteur binaire pBIN 19 (Bevan, 1984, Nucl. Acids Res., 12, 8711-8721), ouvert à l'aide des endonucléases Hind III et EcoRI. Ce vecteur porte deux gènes de résistance à la kanamycine, l'un pouvant s'exprimer dans les bactéries, l'autre situé immédiatement en amont du gène recombinant complet pouvant être transféré aux cellules végétales.

Le vecteur obtenu, appelé pBR 20, est cloné dans la souche *E. coli* HB 101 (Clontech).

### 2) Transformation d'Agrobacterium tumefaciens

La transformation de la souche d'*Agrobacterium tumefaciens*LBA 4404 (Clontech) est réalisée selon la méthode de congélation-décongélation décrite dans Plant Molecular Biology Manual (Gelvin et al, op. cité) (résumé en section 14) à partir de 1 mg de plasmide pBR20.

### 3) Transformation du tabac

Du tabac *Nicotiana tabacum* cultivé in vitro a été infecté par *Agrobacterium tumefaciens* contenant le plasmide pBR 20 selon la procédure de Horsch et al., bien connue de l'homme du métier (Horsch R.B. et al., 1985 Science 227, 1229-1231), dont les principales étapes sont exposées ci-après.

Des disques de feuilles de plantes axéniques de tabac *Nicotiana tabacum* (variété Wisconsin Havana 38) sont incubés dans une culture d'*A. tumefaciens* hébergeant le plasmide pBR 20. Les disques égouttés sur papier Whatman sont mis en culture sur des milieux de culture en boites de Pétri afin de multiplier les cellules transformées de façon à obtenir des cals. Ces cals sont ensuite transférés sur du milieu contenant de la céfotaxime à 500 mg/ml destinée à décontaminer les tissus végétaux (élimination des *Agrobacterium tumefaciens*) et de la kanamycine à 100 mg/ml pour sélectionner le matériel transgénique.

### Mise en évidence de l'expression de la protéine à activité endochitinase dans les tabacs transgéniques

### a) Préparation des extraits bruts de protéines de tabac transformé

Les extraits bruts de protéines ont été préparés à partir de différents tissus de la plante (racine, tige, feuille, etc ...). Les fragments de tissus ont été congelés dans l'azote liquide, réduits en poudre et stockés à -20°C. La poudre a été extraite à 4°C en présence d'un tampon acétate d'ammonium 0,1 M pH 5,2 et soumise à une centrifugation à 10 000 g. La concentration des protéines totales a été déterminée sur les surnageants, appelés ci-après les extraits bruts de protéines en suivant la technique de Bradford (Bradford, M.M., 1976 Anal. Biochem., 72. 248-254).

### b) Mise en évidence de la chitinase hybride par immuno-empreinte (Western Blot)

On soumet les extraits bruts de protéines à un Western blot, technique bien connue de l'homme de l'art et décrite par H. Towbin et al. (Proc. Ntl. Acad. Sci. USA, 76, 1979, 4350-4354).

L'immunodétection de la protéine d'intérêt se réalise grâce à un immunsérum contenant des anticorps polyclonaux reconnaissant la protéine hybride à activité chitinase (cf. EP-493 581, section 5).

Le complexe antigène-anticorps est ensuite révélé à l'aide d'un système streptavidine-biotine conjugué à la phosphatase alcaline avec le kit RPN 23 d'Amersham ("Blotting détection kit"), utilisé selon les indications du fabricant.

L'empreinte obtenue montre, pour les feuilles de plantes de tabac transformées par le plasmide pBR 20, la présence d'une protéine de poids moléculaire apparent d'environ 26 ± 6kDa reconnue par les anticorps polyclonaux et absente des feuilles des plantes de tabac témoins. Cette protéine a le même poids moléculaire apparent que la protéine hybride à activité chitinase décrite dans la demande EP-493 581.

### Section 26 : Localisation des séquences minimales du promoteur 246C responsables des caractéristiques décrites

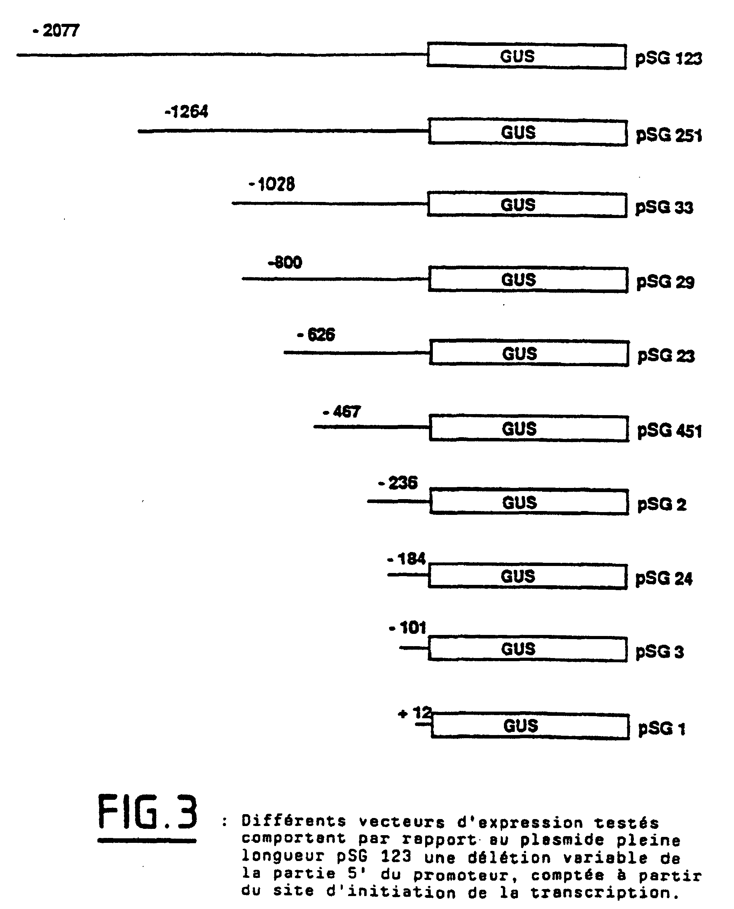

A partir du vecteur pSG123, associant le promoteur du gène 246C au gène de la β-glucuronidase, différentes délétions ont été effectuées dans la région 5' de ce promoteur. Celles-ci ont été obtenues soit par utilisation d'enzymes de restriction et/ou de la nucléase Exo3. L'extension précise des délétions a été déterminée par séquençage selon la méthode de Sanger. La figure 3 présente les différents vecteurs obtenus à l'aide de ces délétions de la partie 5' du promoteur, comptées à partir du site d'initiation de la transcription en position 2068.

### a. Etude de la force du promoteur 246C par expression transitoire dans des protoplastes de tabac.

Ces vecteurs ont été utilisés en expression transitoire sur des protoplastes de tabac qui ne reçoivent aucun effecteur. L'analyse des résultats montre que l'expression maximale est obtenue avec les vecteurs pSG 251 et pSG 33. atteignant 30 000 pmol de méthylumbellifèrone formée/min/mg protéine. Des délétions plus importantes effectuées dans ce promoteur (correspondant aux vecteurs PSG29, pSG23, pSG451, pSG2, pSG24, pSG3, pSG1 ont pour conséquence une réduction de l'expression de la glucuronidase d'autant plus grande que la délétion est importante (tableau ci-dessous).

| | |
|---|---|
| Délétions successives de pSG123 | Activité GUS (pMoles/min/mg) |
| Témoin | 0 |
| pBI221 | ≈ 1000 |
| pSG123 | 5000 |
| pSG251 | 29000 |
| PSG33 | 31000 |
| PSG29 | 26000 |
| PSG23 | 22000 |
| pSG451 | 10000 |
| pSG2 | 4000 |
| PSG14 | 1000 |
| pSG3 | 0 |
| pSG1 | 0 |

Les vecteurs pSG 251 et pSG 33 correspondant respectivement aux promoteurs comprenant la séquence (B) [vecteur pSG 33] et la séquence (C) [vecteur pSG 251] respectivement.

b. Etude de la force du promoteur par expression stable de constructions chimériques renfermant des promoteurs délétés, dans des tabacs transgéniques.

Pour chacun des vecteurs décrits par la figure 3 le gène chimérique associant le promoteur (complet ou tronqué) à la partie codante de la glucuronidase et le terminateur NOS a été purifié sur gel d'agarose après coupure par des endonucléases de restriction Hind III et EcoRI. Dans chacun des cas. le gène chimérique a été introduit et ligué dans le vecteur binaire pBIN19 (Clontech) préalablement ouvert aux sites Hind III et EcoRI (Section 13).

Le tabac, Nicotiana tabacum cultivé in vitro a été infecté par *Agrobactérium tumefaciens* contenant les différentes constructions décrites ci-dessus. La procédure suivie est celle décrite à la Section 15.

L'activité glucuronidase est la moyenne des mesures effectuées sur 10 à 20 transformants indépendants. En l'absence d'inducteur, l'activité glucuronidase de base des différents génotypes n'est pas sensiblement affectée par les délétions pour les constructions allant de pSG251 à pSG451 : elle est sensiblement identique à celle de génotypes renfermant la construction pSG123 (fig.3). Par contre, pour les constructions pSG2, pSG24, pSG3, pSG1, l'expression est d'autant plus faible que la longueur du promoteur est courte : les expressions pour pSG3 et pSG1 étant nulles.

En présence d'inducteurs bactériens (voir section 17), les plantes contenant les constructions pSG251 et pSG33 ont une expression stimulée par un facteur 3 par rapport à la construction pSG123. Ceci indique que la partie délétée correspondant à la séquence D [SEQ. ID No. 6] contient une séquence diminuant l'inductibilité du promoteur 246 C par contre la séquence B [SEQ. ID No. 4] seul, ou en présence de la séquence C [SEQ. ID No. 5] autorise une inductibilité supérieure à celle de la séquence du promoteur 246 C (séquences B+C+D).

L'expression est par contre inchangée pour les génotypes contenant les constructions pSG29 et pSG23 par rapport aux génotypes renfermant les constructions pSG123.

Pour les génotypes contenant les constructions pSG451, pSG2, pSG24, pSG3, pSG1, l'inductibilité est systématiquement inférieure à celles des génotypes renfermant la construction pSG123 : elle est d'autant plus faible que le promoteur est court et s'annule pour les plantes renfermant les constructions pSG3 et psG1.

Ces résultats indiquent que la séquence D [SEQ. ID No. 6] comporte une information de type "silencer" qui inhibe partiellement l'inductibilité du promoteur complet (séquences B+C+D).

## Revendications

1. Promoteur végétal, **caractérisé en ce qu'**il comprend la séquence (B) [SEQ ID NO : 4] ci-après : ou une séquence ayant un degré d'homologie d'au moins 80 % avec la séquence (B).

2. Promoteur selon la revendication 1, **caractérisé en ce qu'**il comprend en amont de la séquence (B), la séquence (C) [SEQ ID NO : 5] ci-après : ou une séquence présentant un degré d'homologie élevé avec la séquence (C).

3. Promoteur selon l'une des revendications 1 ou 2, **caractérisé en ce qu'**il comprend en amont de la séquence (B), la séquence (D) [SEQ ID NO : 6] ci-après : ou une séquence présentant un degré d'homologie élevé avec la séquence (D).

4. Utilisation d'un promoteur selon l'une quelconque des revendications 1 à 3, pour obtenir en situation de stress, la surexpression dans un végétal d'une protéine d'intérêt , cette protéine pouvant être destinée à aider les végétaux à surmonter un état de stress.

5. Cellules végétales, à l'exception des cellules végétales présentes à l'état naturel, microorganismes (bactéries) ou cellules de microorganismes ayant intégré une unité d'expression d'une protéine comprenant le promoteur végétal selon l'une quelconque des revendications 1 à 3.

6. Végétal ou partie de végétal, **caractérisé en ce qu'**il comprend des cellules végétales selon la revendication 5.

7. Semence **caractérisée en ce qu'**elle comprend des cellules végétales selon la revendication 5.

8. Utilisation d'un végétal ou d'une partie de végétal selon les revendications 6 et 7 pour sélectionner des molécules à activité phytosanitaire susceptibles d'induire des réactions de défense naturelles des végétaux contre des organismes phytopathogènes ou phytophages (champignons, bactéries, virus, insectes et nématodes).

## Claims

1. A plant promoter, **characterized in that** it comprises the sequence (B) [SEQ ID NO: 4] below: or a sequence having a degree of homology of at least 80% with the sequence (B).

2. A promoter according to claim 1, **characterized in that** it comprises, upstream from the sequence (B), the sequence (C) [SEQ ID NO:5] below: or a sequence having a high degree of homology with the sequence (C).

3. A promoter according to one of claims 1 or 2 **characterized in that** it comprises, upstream from the sequence (B), the sequence (D) [SEQ ID NO: 6] below: or a sequence having a degree of homology with the sequence (D).

4. Use of a promoter according to any one of claims 1 to 3 for obtaining the superexpression of a protein of interest in a plant in a stress situation, it being possible for the purpose of this protein to be that of helping plants to overcome a stress condition.

5. Plant cells, except plant cells in the natural state, microorganism cells (bacteria) or microorganism cells which have integrated a unit for the expression of a protein comprising the plant promoter according to any one of claims 1 to 3.

6. A plant or part of a plant, **characterized in that** it comprises plant cells according to claim 5.

7. A seed, **characterized in that** it comprises plant cells according to claim 5.

8. Use of a plant or part of a plant according to claims 6 and 7 for selecting molecules with plant protection activity which is capable of inducing natural defense reactions in plants against phytopathogenic or phytophagic organisms (fungi, bacteria, viruses, insects and nematodes).

## Patentansprüche

1. Pflanzenpromotor, **dadurch gekennzeichnet, dass** er die nachstehende Sequenz (B) [SEQ ID NO: 4] oder eine Sequenz mit einem Homologiegrad von mindestens 80 % mit der Sequenz (B) aufweist.

2. Promotor nach Ansrpuch 1, **dadurch gekennzeichnet, dass** er stromaufwärts der Sequenz (B) die nachstehende Sequenz (C) [SEQ ID NO: 5]: oder eine Sequenz mit einem erhöhten Homologiegrad mit der Sequenz (C) aufweist.

3. Promotor nach einem der Ansprüche 1 oder 2, **dadurch gekennzeichnet, dass** er stromaufwärts der Sequenz (B) die nachstehende Sequenz (D) [SEQ ID NO: 6] : oder eine Sequenz mit einem erhöhten Homologiegrad mit der Sequenz (D) aufweist.

4. Verwendung eines Promotors nach einem der Ansprüche 1 bis 3 zur Erzielung einer Überexpression eines Proteins von Interesse in einer Pflanze in einer Stress-Situation, wobei dieses Protein zur Unterstützung von Pflanzen bei der Überwindung eines Stress-Zustands bestimmt sein kann.

5. Pflanzenzellen mit Ausnahme von natürlich vorkommenden Pflanzenzellen, Mikroorganismen (Bakterien) oder Zellen von Mikroorganismen mit einer integrierten Expressionseinheit eines Proteins, das den Pflanzenpromotor nach einem der Ansprüche 1 bis 3 enthält.

6. Pflanze oder Pflanzenteil, **dadurch gekennzeichnet, dass** sie bzw. er Pflanzenzellen nach Anspruch 5 enthält.

7. Samen, **dadurch gekennzeichnet, dass** er Pflanzenzellen nach Anspruch 5 enthält.

8. Verwendung einer Pflanze oder eines Pflanzenteils nach den Ansprüchen 6 und 7 zur Selektionierung von Molekülen mit phytosanitärer Aktivität, die natürliche Verteidigungsreaktionen von Pflanzen gegen phytopathogene oder phytophage Organismen (Pilze, Bakterien, Viren, Insekten und Nematoden) induzieren können.
